# EUROPÄISCHE PATENTSCHRIFT

(11) **EP 2 550 352 B1**
(45) Veröffentlichungstag und Bekanntmachung des Hinweises auf die Patenterteilung: **11.11.2015**
(21) Anmeldenummer: 11709652.9
(22) Anmeldetag: 21.03.2011
(51) Int. Cl.: C12M 1/00, C12M 1/02, C12M 1/12, C12N 5/00

(54) **SUBSTRAT, KULTIVIERUNGSEINRICHTUNG UND KULTIVIERUNGSVERFAHREN FÜR BIOLOGISCHE ZELLEN**
SUBSTRATE, CULTURE FACILITY AND CULTURE METHOD FOR BIOLOGICAL CELLS
SUBSTRAT, DISPOSITIF DE CULTURE ET PROCÉDÉ DE CULTURE DE CELLULES BIOLOGIQUES

(30) Priorität: 22.03.2010 DE 102010012254
(43) Veröffentlichungstag der Anmeldung: 30.01.2013
(73) Patentinhaber: Fraunhofer-Gesellschaft zur Förderung der angewandten Forschung e.V., 80686 München (DE)
(72) Erfinder: DUSCHL, Claus, 10115 Berlin (DE); LANKENAU, Andreas, 6343 Rotkreuz (CH); LUTZ, Jean-Francois, 77694 Kehl (DE); LASCHEWSKY, André, 14469 Potsdam (DE); WISCHERHOFF, Erik, 14469 Potsdam (DE); FUHR, Günter, R., 13187 Berlin (DE); BIER, Frank, 14482 Potsdam (DE)
(74) Vertreter: Hertz, Oliver
(86) Internationale Anmeldenummer: PCT/EP2011/001393
(87) Internationale Veröffentlichungsnummer: WO 2011/116921

(56) Entgegenhaltungen:
- EP-A1- 1 942 179
- WO-A2-2004/011669
- CHENG X ET AL: "Novel cell patterning using microheater-controlled thermoresponsive plasma films", JOURNAL OF BIOMEDICAL MATERIALS RESEARCH - PART A 20040801 JOHN WILEY AND SONS INC. US, Bd. 70A, Nr. 2, 1. August 2004 (2004-08-01), Seiten 159-168, XP002636205,
- ERNST OLIVER ET AL: "Control of cell detachment in a microfluidic device using a thermo-responsive copolymer on a gold substrate", LAB ON A CHIP, Bd. 7, Nr. 10, 2007, Seiten 1322-1329, XP002636206,

## Beschreibung

Die Erfindung betrifft ein thermoresponsives Substrat zur Aufnahme biologischer Zellen, insbesondere ein Substrat, dessen Oberflächeneigenschaften in Abhängigkeit von der Temperatur veränderlich sind. Des Weiteren betrifft die Erfindung eine Kultivierungseinrichtung für biologische Zellen, die mit mindestens einem derartigen Substrat ausgestattet ist. Des Weiteren betrifft die Erfindung ein Verfahren zur gezielten Anordnung biologischer Zellen unter Verwendung des Substrats und der Kultivierungseinrichtung, insbesondere ein Verfahren zur Kultivierung biologischer Zellen. Anwendungen der Erfindung sind bei der *in* vitro-Kultivierung biologischer Zellen gegeben.

Es ist bekannt, in der regenerativen Medizin oder in der Implantationsmedizin biologische Zellen, wie einzelne Zellen, Zellschichten, Zellgruppen, Gewebe oder organoide Zellstrukturen, für die Nachbildung physiologischer Funktionen oder für Implantationszwecke zu verwenden. Aus der Praxis ist bekannt, dass Eigenschaften biologischer Zellen durch deren unmittelbare Umgebung (Mikroumgebung) beeinflusst werden. Beispielsweise hängen das Wachstum und die Differenzierung von Zellen vom Vorhandensein von Signalfaktoren in der Mikroumgebung ab. Da die Mikroumgebung biologischer Zellen im lebenden Organismus durch weitere biologische Zellen gebildet wird, besteht ein Interesse, auch bei der *in* vitro-Kultivierung eine aus Zellen gebildete Mikroumgebung künstlich herzustellen und insbesondere Zellen verschiedener Zelltypen mit einer vorbestimmten geometrischen Anordnung zur Nachbildung realer Bedingungen im lebenden Organismus zu kombinieren.

Aus der Zellbiologie sind zahlreiche Techniken zur geometrischen Anordnung biologischer Zellen bekannt, wobei die Zellen zum Beispiel im suspendierten Zustand mit dielektrophoretischen Kräften oder mit optischen Pinzetten oder im adhärenten Zustand mit mechanischen Werkzeugen manipuliert werden. Diese Techniken basieren jedoch meistens auf einer Einzelzellmanipulation, so dass sie für eine effektive zwei- oder dreidimensionale Zellanordnung im adhärenten Zustand unpraktikabel und nur beschränkt anwendbar sind. Es ist ferner möglich, verschiedene Zelltypen im suspendierten Zustand zu mischen und anschließend für eine Cokultivierung auf einem Substrat anzuordnen. Eine genaue und reproduzierbare Einstellung der Mikroumgebung einzelner Zellen ist damit jedoch nicht möglich. Des Weiteren ist aus der Praxis bekannt, Gewebeteile zu kombinieren oder einzelne biologische Zellen oder Zellgruppen in einem Gewebe zu applizieren. In diesen Fällen wird die Mikroumgebung durch das Gewebe vorgegeben, so dass die Kultivierungsbedingungen der Mikroumgebung nur beschränkt variiert werden können.

Des weiteren sind thermoresponsive Polymere bekannt. Ein thermoresponsives Polymer zeichnet sich dadurch aus, dass es eine Schalttemperatur (LCST, "lower critical solution temperature") in wässrigen Medien aufweist. Wässrige Medien sind z. B. reines Wasser, kommerziell erhältliche Pufferlösungen, Zellkulturmedien oder Mischungen von Wasser mit organischen Lösungsmitteln. Unterhalb der Schalttemperatur sind wässrige Lösungen thermoresponsiver Polymere einphasig, darüber zweiphasig. Wenn thermoresponsive Polymere an Oberflächen immobilisiert werden, so vollziehen sie in wässrigen Medien bei Überschreiten der Schalttemperatur einen Phasenübergang (Konformationsübergang): sie sind unterhalb der Schalttemperatur stärker hydratisiert als oberhalb.

Es ist bekannt, dass die Adhäsion auf Substraten, die mit dem thermoresponsiven (thermosensitiven) Polymer Poly-(N-isopropyl-acrylamid) ("PNIPam") oder Derivaten davon beschichtet sind und die temperaturabhängige Hydratisierung aufweisen, gezielt in Abhängigkeit von der Temperatur beeinflusst werden kann (siehe N. Yamada et al. in "Makromol. Chem." 11 (1990) 571; C. Williams et al. in "Adv. Mater." 21 (2009) 2161-2164, O. Ernst et al. in "Lab Chip" 7 (2007) 1322). Diese Eigenschaft wurde auch an Polyethylenglykol (PEG)-basierten Polymeren gezeigt (siehe E. Wischerhoff et al. in "Angew. Chem." 47 (2008) 5666).

Von Y. Yamanishi et al. wird in "IEEE Transactions on Nanobioscience" (8(4), 2009; 312-317) die Anwendung von PNIPam zur zeitweiligen Lokalisierung biologischer Zellen in einem fluidischen Mikrosystem beschrieben. Das fluidische Mikrosystem wird von einer PNIPam-Lösung durchströmt, in der Zellen suspendiert sind. An einem Heizelement kann die Lösung unterhalb einer so genannten Mikrobrücke geliert und in einen festen Zustand überführt werden, in dem die Zellen fixiert sind und untersucht werden können. Die von Y. Yamanishi et al. beschriebene Technik ist auf die Handhabung suspendierter und in einem Gel eingeschlossener Zellen beschränkt und für die Untersuchung von Zellen im adhärenten Zustand ungeeignet.

In WO 2004/011669 A2 und in der Publikation von X. Cheng et al. ("Journal of Biomedical Materials Research - Part A" 70, 2004, 159) wird ein Substrat zur Aufnahme von Zellen beschrieben, das aus thermoresponsivem Polymer hergestellt und mit Heizelementen ausgestattet ist. In EP 1 942 179 A1 wird ein Substratchip mit einer Schicht aus einem thermoresponsiven Polymer beschrieben, wobei die Schicht eine Vielzahl mikroskopisch kleiner Löcher aufweist. Durch eine Erwärmung oder Abkühlung der Schicht können die Maße der Löcher verändert werden, um biologische Materialien in den Löchern einzufangen oder aus diesen freizugeben.

Der Aufgabe der Erfindung ist es, verbesserte Vorrichtungen für die in vitro-Kultivierung biologischer Zellen bereitzustellen, mit denen Nachteile herkömmlicher Kultivierungs- oder Untersuchungstechniken überwunden werden können. Die Aufgabe der Erfindung ist es insbesondere, Vorrichtungen bereitzustellen, die eine gezielte, schonende Anordnung biologischer Zellen mit frei wählbaren Umgebungsbedingungen ermöglichen. Die Aufgabe der Erfindung ist des weiteren, verbesserte Verfahren zur Kultivierung biologischer Zellen bereitzustellen, mit denen Nachteile herkömmlicher Techniken vermieden werden. Erfindungsgemäße Verfahren sollen insbesondere geeignet sein, gezielt Mikroumgebungen für die Kultivierung, z. B. in Bezug auf Auswahl von Zelltypen, Zellzahlen und Geometrien der Zellanordnung, herzustellen.

Diese Aufgaben werden durch ein Substrat, eine Kultivierungseinrichtung und ein Verfahren mit den Merkmalen der unabhängigen Ansprüche gelöst. Vorteilhafte Ausführungsformen und Anwendungen der Erfindung ergeben sich aus den abhängigen Ansprüchen.

Gemäß einem ersten Gesichtspunkt der Erfindung wird ein Substrat bereitgestellt, das zur Aufnahme biologischer Zellen eingerichtet ist und insbesondere einen Substratkörper mit einer Trägerfläche aufweist, auf der ein thermoresponsives Polymermaterial angeordnet ist. Erfindungsgemäß sind Widerstands-Heizelemente und Peltier-Kühlelemente (Temperierungselemente, Temperatureinstelleinrichtung) vorgesehen, die zur Temperierung von mindestens einem jeweils zugehörigen Segment (Teilbereich, Abschnitt) der Trägerfläche angeordnet sind. Mit den Temperierungselementen ist die Temperatur der eine Segmente der Trägerfläche lokal spezifisch (lokal abgegrenzt) und von der Temperatur umgebender Segmente abweichend einstellbar. Jedes Temperierungselement ist angeordnet, die Temperatur im zugehörigen Segment der Trägerfläche in einem Temperaturbereich zwischen einer Temperatur oberhalb einer kritischen Phasenübergangs-Temperatur (Schalttemperatur) des thermoresponsiven Polymermaterials und einer Temperatur unterhalb der Schalttemperatur des thermoresponsiven Polymermaterials einzustellen.

Das erfindungsgemäße Substrat mit den lokal spezifisch temperierbaren Segmenten der Trägerfläche ermöglicht vorteilhafterweise die Bereitstellung von Segmenten mit verschiedenen Adhäsionseigenschaften für biologische Zellen. Erfindungsgemäß wird eine thermisch schaltbare Oberfläche bereitgestellt, die durch die lokal wirkenden Heiz- und Kühlelemente reversibel und schaltbar eine ortsaufgelöste Kontrolle der Zellanhaftung ermöglicht. Damit können in mehreren Schritten an bestimmten Orten Zellen entfernt oder hinzugefügt werden, so dass schließlich ein definiert strukturierter Zellverband entsteht. Allein durch die Temperierung der Segmente, insbesondere die Einstellung der Temperatur oberhalb oder unterhalb der Schalttemperatur, kann bestimmt werden, ob ein Segment für eine Adhäsion biologischer Zellen geeignet ist (Adhäsions-Segment) oder ein Segment für die Adhäsion biologischer Zellen ungeeignet ist (Leer-Segment). Die Temperierungselemente sind einzeln steuerbar, so dass die Funktion der Segmente als Adhäsion- oder Leer-Segmente frei wählbar und im Zeitverlauf veränderlich ist. Vorteilhafterweise ermöglicht dies die gezielte Adhäsion biologischer Zellen mit einer vorbestimmten geometrischen Anordnung. Durch die lokal spezifische Einstellung der Adhäsionseigenschaften des Substrats können gezielt Zellanordnungen geschaffen und somit Mikroumgebungen für adhärente Zellen nachgebildet werden.

Das erfindungsgemäße Substrat ist ein Kultivierungssubstrat für biologische Zellen. Das Substrat ist zur Aufnahme biologischer Zellen und zur Bereitstellung von physiologischen Kultivierungsbedingungen konfiguriert. Das Substrat ist insbesondere zur Aufnahme der Zellen in einem flüssigen Kultivierungsmedium eingerichtet, d.h. die Trägerfläche ist geeignet, mit dem Kultivierungsmedium bedeckt zu werden. Der Substratkörper kann aus einem festen Material hergestellt sein, das starr oder nachgiebig (biegsam) ist. Das Material des Substratkörpers ist vorzugsweise temperaturstabil und insbesondere nicht thermoresponsiv. Die Trägerfläche ist vorzugsweise eine ebene Fläche, kann jedoch alternativ gekrümmt gebildet sein.

Gemäß einem zweiten Gesichtspunkt der Erfindung wird eine Kultivierungseinrichtung für biologische Zellen bereitgestellt, die mindestens ein erfindungsgemäßes Substrat gemäß dem ersten Gesichtspunkt der Erfindung enthält und zur Aufnahme eines Kultivierungsmediums eingerichtet ist. Das Kultivierungsmedium bedeckt die Trägerfläche des mindestens einen Substrats. Vorteilhafterweise erfüllt das Kultivierungsmedium mehrere Funktionen. Erstens dient das Kultivierungsmedium in Abhängigkeit von der Funktion des mindestens einen Temperierungselements als Wärmequelle und/oder Wärmesenke. Des Weiteren können mit dem Kultivierungsmedium gezielt Zellen zur Trägerfläche des Substrats zugeführt werden oder von der Trägerfläche abgelöst (abgespült) werden. Schließlich kann das Kultivierungsmedium auch zur Zufuhr von Nährstoffen zu den Zellen verwendet werden.

Die Kultivierungseinrichtung umfasst allgemein ein Gerät, das zur Kultivierung biologischer Zellen eingerichtet ist und insbesondere ein Kultivierungsgefäß zur Aufnahme des Kultivierungsmediums und eine Fluidikeinrichtung zur Zu- und/oder Abfuhr des Kultivierungsmediums in das Kultivierungsgefäß aufweist. Die Größe, Form und Gestaltung dieser Komponenten kann in Abhängigkeit von den konkreten Anwendungsbedingungen gewählt werden. Das mindestens eine erfindungsgemäße Substrat ist so angeordnet, dass die Trägerfläche zur Aufnahme der biologischen Zellen im Kultivierungsgefäß frei liegt. Das mindestens eine Substrat kann mit der Kultivierungseinrichtung fest verbunden sein, z. B. den Boden des Kultivierungsgefäßes bilden, oder von der Kultivierungseinrichtung lösbar sein, z. B. ein in das Kultivierungsgefäß einlegbares Teil darstellen. Gemäß einer weiteren Alternative können die Temperierungselemente mit dem Kultivierungsgefäß fest verbunden sein, während der Substratkörper ein von dem Kultivierungsgefäß trennbares Teil ist. Typischerweise ist das Kultivierungsmedium flüssig. Für spezielle Kultivierungsaufgaben kann die Bildung einer gasförmigen Umgebung der Zellen vorgesehen sein.

Gemäß einem dritten Gesichtspunkt der Erfindung wird ein Verfahren zur Anordnung biologischer Zellen auf dem erfindungsgemäßen Substrat in der erfindungsgemäßen Kultivierungseinrichtung bereitgestellt. Erfindungsgemäß ist vorgesehen, dass die Temperatur der Segmente der Trägerfläche gezielt so eingestellt wird, dass ein vorbestimmtes Adhäsionsmuster gebildet wird, und eine Ablage biologischen Zellen in mindestens einem der Segmente erfolgt, dessen Temperatur oberhalb der Schalttemperatur des thermoresponsiven Polymermaterials ist. Vorteilhafterweise ermöglicht das erfindungsgemäße Kultivierungsverfahren eine gezielte Ablage der Zellen gemäß dem vorgegebenen Adhäsionsmuster. Die Schaffung von Mikroumgebungen für die *in* vitro-Kultivierung wird mit hoher Effektivität für eine frei wählbare Anzahl von Zellen ermöglicht.

Vorteilhafterweise sind zahlreiche Varianten des erfindungsgemäßen Substrats verfügbar, die einzeln oder in Kombination in Abhängigkeit von den konkreten Anwendungsbedingungen gewählt werden. So umfassen die Temperierungselemente selektiv betätigbare Widerstands-Heizelemente. Bei Betätigung des Heizelements wird die Temperatur des zugehörigen Segments erhöht, so dass sich in dem Segment die gewünschte Adhäsionsfähigkeit für Zellen ergibt. In Segmenten mit inaktiven Heizelementen hingegen können die Zellen nicht adhärieren. Zusätzlich umfassen die Temperierungselemente selektiv betätigbare Peltier-Kühlelemente, die entsprechend bei Betätigung die Adhäsionsfähigkeit für Zellen unterbinden und im inaktiven Zustand die Adhäsionsfähigkeit ergeben.

Erfindungsgemäß sind Heiz- und Kühlelemente kombiniert, um eine Temperaturverteilung der Trägerfläche einzustellen und um biologische Zellen von einem Zelltyp oder mehreren Zelltypen gezielt in vorbestimmten Segmenten anzusiedeln.

Gemäß der Erfindung sind die Temperierungselemente mit dem Substratkörper fest verbunden. Die Temperierungselemente können zum Beispiel auf einer Rückseite des Substratkörpers, das heißt auf der Rückseite befestigt, in das Innere des Substratkörpers eingebettet und/oder auf der Trägerfläche des Substratkörpers angeordnet sein.

Gemäß der Erfindung ist eine Vielzahl von Temperierungselementen vorgesehen. Mit den Temperierungselementen sind Segmente der Trägerfläche einzeln, vorzugsweise oberhalb oder unterhalb der Schalttemperatur des thermoresponsiven Polymermaterials, temperierbar. Gegenüber der Verwendung eines einzigen Temperierungselement wird vorteilhafterweise die Variabilität bei der Einstellung einer Temperaturverteilung der Trägerfläche erhöht.

Vorteilhafterweise kann die Größe und Form der Temperierungselemente und deren geometrische Anordnung frei gewählt und insbesondere an die konkreten Anwendungsbedingungen angepasst werden. Wenn gemäß einer bevorzugten Ausführungsform der Erfindung die Temperierungselemente eine Matrixanordnung bilden, wobei die Temperierungselemente in geraden Reihen und Spalten angeordnet sind, wird vorteilhafterweise eine hohe Flexibilität bei der Einstellung des Adhäsionsmusters der Trägerfläche erzielt. Alternativ können die Temperierungselemente eine Anordnung in Form gerader oder gekrümmter Streifen, z. B. eine Ringanordnung, insbesondere eine konzentrische Ringanordnung bilden. Diese Varianten können für die Anpassung des Adhäsionsmusters an Migrationseigenschaften der adhärenten Zellen auf dem Substrat von Vorteil sein. Alternativ oder zusätzlich kann eine unregelmäßige Anordnung von Temperierungselementen vorgesehen sein. Diese kann Vorteile für die Beeinflussung der Temperaturverteilung auf der Trägerfläche haben.

Gemäß einer weiteren Ausführungsform der Erfindung kann das Substrat mit einer Elektrodeneinrichtung ausgestattet sein. Die Elektrodeneinrichtung umfasst mindestens ein einzelnes Elektrodenelement, vorzugsweise eine Vielzahl von Elektrodenelementen, die mit hochfrequenten elektrischen Spannungen beaufschlagt werden können, um in ihrer Umgebung, insbesondere oberhalb der Trägerfläche dielektrophoretisch wirkende Kräfte zu erzeugen. Mittels negativer oder positiver Dielektrophorese können im Kultivierungsmedium suspendierte biologische Zellen zu den Segmenten, deren Temperatur oberhalb der Schalttemperatur ist (Adhäsions-Segmente) gezogen oder von den übrigen Segmenten abgestoßen werden. Die Zahl und die geometrische Anordnung der Elektrodenelemente sind vorzugsweise gleich der Zahl und Anordnung der Temperierungselemente gewählt. Jedem Temperierungselement ist ein Elektrodenelement zur Erzeugung lokal wirkender dielektrophoretischer Kräfte zugeordnet.

Vorteilhafterweise kann auch der Substratkörper des erfindungsgemäßen Substrats in Abhängigkeit von den konkreten Anwendungsbedingungen angepasst werden. Beispielsweise kann der Substratkörper eine Hohlform haben, auf deren Innenseite die Trägerfläche gebildet ist. Der Substratkörper wird durch eine Hohlleitung, wie z. B. eine Kapillare, gebildet, in deren Inneren der Kultivierungsraum zur Aufnahme des Kultivierungsmediums vorgesehen ist. Die Temperierungselemente können axial und/oder azimuthal in Bezug auf die Längsachse der Hohlform verteilt angeordnet sein. Gemäß einer weiteren Variante kann der Substratkörper mit einer gekrümmten Trägerfläche, z. B. in Form mindestens eines Napfes gebildet sein. Diese Ausführungsform der Erfindung hat besondere Vorteile für die Nachbildung so genannter Zellnischen, insbesondere Stammzellnischen. Für eine parallele Kultivierung einer Vielzahl von Zellen oder Zellgruppen kann es von Vorteil sein, wenn der Substratkörper eine Platte umfasst, in der eine Vielzahl von Näpfen angeordnet sind.

Gemäß einer weiteren Variante der Erfindung kann der Substratkörper flüssigkeitsdurchlässig sein. Der Substratkörper kann z. B. aus einem porösen Material, wie z. B. einem porösen Kunststoff oder einer porösen Keramik hergestellt sein. Vorteilhafterweise ermöglicht dies, dass Kultivierungsmedium zur Versorgung der adhärenten Zellen durch den Substratkörper hindurchtritt.

Wenn gemäß einer bevorzugten Ausführungsform der Erfindung der Kultivierungsraum zur Aufnahme des Kultivierungsmediums zwischen zwei zueinander weisenden Substraten gebildet ist, können sich Vorteile für die gezielte dreidimensionale Anordnung biologischer Zellen ergeben. Die Trägerflächen der Substrate sind einander gegenüber liegend mit einem Abstand angeordnet. Die auf den Trägerflächen gebildeten Anordnungen biologischer Zellen können durch Wachstum und/oder Zufuhr von Zellen wachsen, bis sie den Abstand überbrücken und sich verbinden. Mit der Bereitstellung des zweiten Substrat wird vorteilhafterweise ein zusätzliche Freiheitsgrad bei der Gestaltung der Mikroumgebung von Zellen erhalten.

Gemäß einer weiteren vorteilhaften Ausführungsform der Erfindung kann das Kultivierungsgefäß der Kultivierungseinrichtung eine Hohlleitung, wie z. B. eine Kapillare, ein Rohr oder einen Schlauch, umfassen, deren Wand das erfindungsgemäße Substrat bildet. Die Trägerfläche ist die Innenfläche der Hohlleitung. Das mindestens eine Temperierungselement ist vorzugsweise auf der Außenseite der Hohlleitung verteilt angeordnet. Das Innere der Hohlleitung bildet den Kultivierungsraum zur Aufnahme des Kultivierungsmediums.

Vorteilhafterweise ist die Kultivierungseinrichtung miniaturisierbar. Sie kann ein fluidisches Mikrosystem umfassen, dessen Bestandteil das erfindungsgemäße Substrat ist. Das fluidische Mikrosystem umfasst eine Anordnung von Fluidkanälen zur Aufnahme des Kultivierungsmediums und gegebenenfalls weiterer Flüssigkeiten und Fluidikelemente zur Steuerung des Flüssigkeitstransports in den Fluidkanälen. Das Substrat ist in einer Wand, z. B. einem Boden, von mindestens einem der Fluidkanäle integriert.

Gemäß einer weiteren vorteilhaften Ausführungsform der Erfindung kann die Kultivierungseinrichtung eine Vielzahl von Kultivierungsstationen aufweisen, zwischen denen das Substrat beweglich ist und die für die Einstellung verschiedener Kultivierungsbedingungen eingerichtet sind. Die Kultivierungseinrichtung mit einer Vielzahl von Kultivierungsstationen ermöglicht vorteilhafterweise eine kontinuierliche Verfahrensführung. Des Substratkörper des erfindungsgemäßen Substrats hat hierzu vorzugsweise die Form eines Substratstreifens, der für einen Durchlauf durch die Kultivierungsstationen eingerichtet ist. Die einzelnen Schritte des erfindungsgemäßen Verfahrens können an den Kultivierungsstationen getrennt durchgeführt werden.

Gemäß einer bevorzugten Ausführungsform das erfindungsgemäßen Verfahrens ist eine Variation des Adhäsionsmusters der Segmente vorgesehen, deren Temperatur oberhalb der Schalttemperatur des thermoresponsiven Polymermaterials gewählt ist. Hierzu wird die Temperatur der Segmente der Trägerfläche in Abhängigkeit von der Zeit so variiert, dass die Zellen von einem oder mehreren Zelltypen aufeinander folgend in verschiedenen Segmenten anhaften. Beispielsweise können Zellen von einem ersten Zelltyp in einem ersten Schritt auf einer vorbestimmten Gruppe von Segmenten abgelegt werden, während der übrigen Segmente frei bleiben. In einem zweiten Schritt können die freien Segmente aus dem ersten Schritt in Segmente mit einer Adhäsionsfähigkeit umgeschaltet werden. Wenn dann mit dem Kultivierungsmedium Zellen von einem zweiten Zelltyp zugeführt werden, so haften diese in den beim ersten Schritt frei gebliebenen Segmenten an. Wenn es sich um Zellen handelt, die Monolagen bilden und nicht oder nur mit verminderter Bindungsrate auf vorhandenen adhärenten Zellen anhaften, so haften die Zellen des zweiten Zelltyps ausschließlich oder vorrangig auf den Segmenten an, die im ersten Schritt frei geblieben sind. Falls eine Bedeckung aller Segmente durch den zweiten Zelltyp erzielt wird, können sich dreidimensionale Zellanordnungen bilden.

Alternativ oder zusätzlich kann die Adhäsionsfähigkeit von Segmenten geschaltet werden, um bereits adhärent angeordnete Zellen in den jeweiligen Segmenten festzuhalten oder abzulösen (abzutrennen), z. B. mit dem Kultivierungsmedium abzuspülen.

Vorteilhafterweise können mit dem erfindungsgemäßen Verfahren Zellen verschiedener Zelltypen in zueinander benachbarten Segmenten gezielt abgelegt werden. Dies ermöglicht die genaue und reproduzierbare Konstruktion von Mikroumgebungen, z. B. für Kultivierungs- oder Testzwecke.

Die zeitliche Variation der Temperatur der Segmente kann erfindungsgemäß vorgesehen sein, während das Substrat in der Kultivierungseinrichtung ortsfest positioniert ist. Alternativ kann eine Bewegung des Substrats zwischen den verschiedenen Kultivierungsstationen der Kultivierungseinrichtung vorgesehen sein. An den Kultivierungsstationen werden verschiedene Kultivierungsbedingungen bereitgestellt, wie z. B. die Zufuhr von bestimmten Nährstoffen oder Signalfaktoren und/oder die gezielte Ablage von Zellen zur Konstruktion der Mikroumgebung.

Erfindungsgemäß kann vorgesehen sein, dass das Substrat mit den Temperierungselementen zu den Kultivierungsstationen beweglich ist. In diesem Fall werden Temperierungselemente an den Kultivierungsstationen mit passenden Versorgungseinrichtungen, z. B. mit einer Stromversorgung oder mit einer Arbeitsmedium-Versorgung gekoppelt. Alternativ kann die erfindungsgemäße Zusammensetzung aus dem Substratkörper und den Temperierungselementen an jeder Kultivierungsstation geschaffen werden. Hierzu sind an jeder Kultivierungsstation Temperierungselemente vorgesehen, mit denen das Substrat an der Kultivierungsstation in thermischen Kontakt gebracht wird.

Besondere Vorteile für die weitere Handhabung der erfindungsgemäß erzeugten zwei- oder dreidimensionalen Zellanordnung ergeben sich, wenn unmittelbar nach der Ablage der biologischen Zellen und gegebenenfalls einer Kultivierung auf dem erfindungsgemäßen Substrat eine Kryokonservierung der Zellen vorgesehen ist. Die Zellen werden in einen gefrorenen Zustand überführt, in dem sie für die gewünschte Anwendung z. B. in der regenerativen Medizin oder in der Implantationsmedizin unverändert erhalten bleiben. Die Kryokonservierung kann z. B. an der letzten Kultivierungsstation der erfindungsgemäßen Kultivierungseinrichtung vorgesehen sein.

Weitere Einzelheiten und Vorteile der Erfindung werden im Folgenden unter Bezug auf die beigefügten Zeichnungen beschrieben. Es zeigen:
- Figur 1:: schematischen Illustrationen bevorzugter Ausführungsformen des erfindungsgemäßen Verfahrens zur gezielten Anordnung biologischer Zellen;
- Figuren 2 und 3:: schematischen Illustrationen weiterer Verfahren zur gezielten Anordnung biologischer Zellen (keine Ausführungsformen der Erfindung);
- Figur 4:: schematischen Illustrationen weiterer bevorzugter Ausführungsformen des erfindungsgemäßen Verfahrens zur gezielten Anordnung biologischer Zellen;
- Figuren 5 und 6:: schematischen Illustrationen von Ausführungsformen der Erfindung, bei denen die Zellen mit dielektrophoretischen Kräften manipuliert werden;
- Figuren 7 und 8:: eine schematische Illustration einer erfindungsgemäßen Kultivierungseinrichtung mit zwei Substraten;
- Figuren 9 bis 11:: weitere Substrate mit Temperierungselementen (keine Ausführungsformen der Erfindung);
- Figuren 12 bis 14:: weitere Ausführungsformen der Erfindung, bei denen die Zellen auf gekrümmten Trägerflächen abgelegt werden;
- Figur 15:: eine schematische Illustration einer kombinatorischen Wechselwirkung von Zellen verschiedener Zelltypen;
- Figuren 16 und 17:: Ausführungsformen der erfindungsgemäßen Kultivierungseinrichtung mit mehreren Kultivierungsstationen;
- Figuren 18 bis 21:: Ausführungsformen der erfindungsgemäßen Kultivierungseinrichtung mit einem fluidischen Mikrosystem; und
- Figur 22:: eine Illustration der Kryokonservierung erfindungsgemäß gebildeter Zellanordnungen.

Bevorzugte Ausführungsformen der Erfindung werden im Folgenden unter beispielhaftem Bezug auf die Gestaltung des Substrats und der Temperierungselemente, insbesondere deren Form, Größe und Anordnung, die Gestaltung der Kultivierungseinrichtung und bevorzugte Varianten des erfindungsgemäßen Verfahrens beschrieben. Einzelheiten der Handhabung der biologischen Zellen und der Steuerung der Adhäsionsfähigkeit der Zellen unter Verwendung von thermoresponsiven Polymeren werden hier nicht beschrieben, da diese an sich aus dem Stand der Technik bekannt sind. Kultivierungsbedingungen, wie z. B. die Zusammensetzung des Kultivierungsmediums oder die Kombination von Zelltypen, und weitere Einzelheiten beim Aufbau der Kultivierungseinrichtung können vom Fachmann in Abhängigkeit von der konkreten Anwendung der Erfindung gewählt werden.

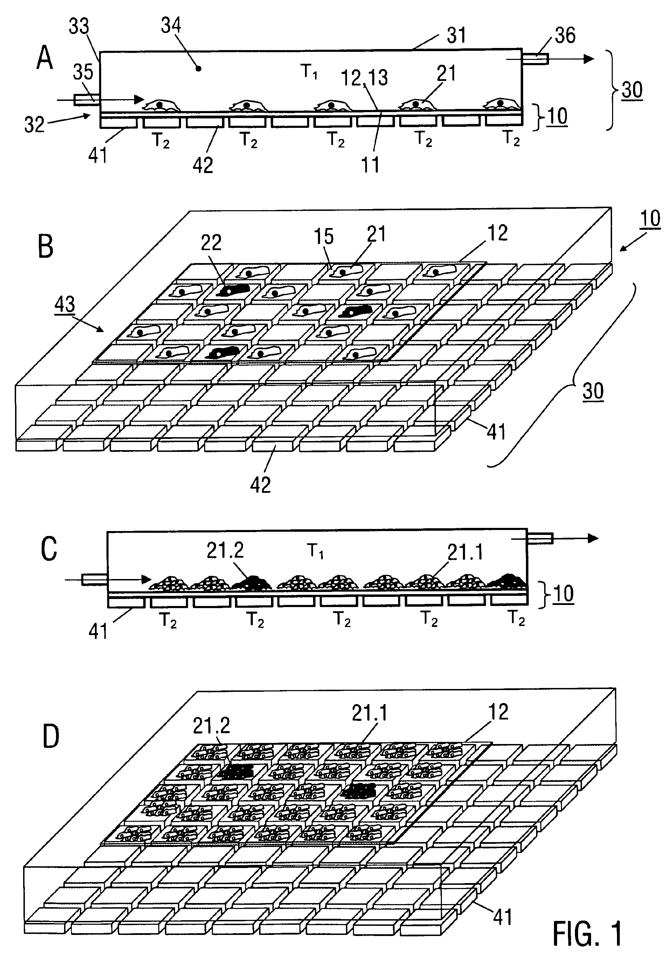

Eine erste Ausführungsform der Erfindung ist in den Figuren 1A bis 1F am Beispiel der gezielten Ablage biologischer Zellen verschiedener Zelltypen auf verschiedenen Segmenten eines Substrats illustriert. Die Anhaftung der Zellen wird durch eine ortsaufgelöste Steuerung lokaler Temperaturveränderungen bewirkt. Die Figuren 1A und 1B zeigen das Substrat 10 als Teil der Kultivierungseinrichtung 30 in schematischen Schnitt- und Perspektivansichten. Das Substrat 10 umfasst den Substratkörper 11, dessen obere Oberfläche die Trägerfläche 12 bildet. Auf der Trägerfläche 12 ist eine Schicht aus thermoresponsivem Polymermaterial 13 gebildet. Der Substratkörper 11 oder mindestens die Trägerfläche 12 ist z. B. aus Glas, Keramik oder Silizium mit einer Dicke z. B. im Bereich von 1 µm bis 1 mm hergestellt. Weitere Materialbeispiele umfassen Kunststoffe, weitere Halbleiter, Metalle, Quarz, Fasern, z. B. Cellulosefasern oder daraus hergestellte Materialien, wie z. B. Papier.

Auf der Unterseite des Substratkörpers 11 sind die Temperierungselemente angeordnet, die Heizelemente 41 und Kühlelemente 42 umfassen. Die Temperierungselemente 41, 42, die eine Matrixanordnung 43 mit geraden Reihen und Spalten bilden, sind z. B. elektrisch betriebene Widerstandselemente und Peltierelemente. Jedes der elektrisch betriebenen Temperierungselemente 41, 42 ist einzeln elektrisch mit einer Versorgungseinrichtung (nicht dargestellt) verbunden, wobei jedes der Temperierungselemente 41, 42 separat betätigbar ist.

Die Temperierungselemente 41, 42 können auf einem gemeinsamen Träger (nicht dargestellt) angeordnet sein, wobei der Substratkörper 11 auf den Temperierungselementen 41, 42 befestigt, z. B. geklebt ist. Alternativ kann der Substratkörper 11 einen Träger für die Temperierungselemente 41, 42 bilden. In diesem Fall können die elektrisch betriebenen Temperierungselemente 41, 42 durch Abscheidungs- und Strukturierungsverfahren auf der Unterseite des Substratkörpers 11 schichtförmig gebildet sein. Gemäß einer weiteren Variante können die Temperierungselemente 41, 42 einen Teil des Substratkörpers 11 bilden. In diesem Fall ist auf den Temperierungselementen 41, 42 eine inerte Schicht, z. B. aus Glas, Metall oder Kunststoff (Dicke im Bereich von 0,1 µm bis 100 µm) vorgesehen, die das thermoresponsive Polymermaterial 13 trägt.

Die Form der Temperierungselemente 41, 42 ist frei wählbar. Beispielsweise können wie dargestellt rechteckige Temperierungselemente 41, 42 oder alternativ andere Formen, wie z. B. kreisförmige Temperierungselemente 41, 42 vorgesehen sein. Eine typische Dimension, z. B. Seitenlänge oder Durchmesser, eines Temperierungselements ist z. B. im Bereich von 10 µm bis 500 µm gewählt. Die Größe der vom Substrat 10 aufgespannten Trägerfläche ist z. B. 1 mm² bis 100 cm² gewählt.

Das auf der Trägerfläche 12 gebildete thermoresponsive Polymermaterial 13 kann wie bei herkömmlichen thermoresponsiven Substraten eine isotrope oder homogene Polymerschicht oder alternativ eine Schicht aus thermoresponsiven Mikrogelen umfassen. Die Dicke des thermoresponsiven Polymermaterials 13 beträgt mindestens 5 nm, vorzugsweise mindestens 20 nm, und weniger als 100 µm, vorzugsweise weniger als 1 µm.

Kultivierungssubstrate, auf deren Oberfläche thermoresponsive Mikrogele angeordnet sind, werden in der Patentanmeldung WO 2011/116922 A2 "Thermoresponsives Substrat mit Mikrogelen, Verfahren zu dessen Herstellung und Kultivierungsverfahren für biologische Zellen" beschrieben, die den selben Prioritätstag wie die vorliegende Patentanmeldung hat.

Das thermoresponsive Polymermaterial 13 zeichnet sich dadurch aus, dass es eine Schalttemperatur (LCST, "lower critical solution temperature") in physiologischem Puffer oder Zellkulturmedium aufweist, die im Bereich von 20 °C bis 40 °C liegt. Oberflächen, die mit dem thermoresponsiven Polymermaterial 13 beschichtet sind, erlauben bei Temperaturen oberhalb der Schalttemperatur, z.B. bei 37 °C, die Anhaftung lebender Zellen. Unterhalb der Schalttemperatur wird die Anhaftung vermindert oder vollständig unterbunden.

Für die Beschichtung der Trägerfläche des Substrates kann eine Vielzahl von thermoresponsiven Polymermaterialien in Betracht gezogen werden, von denen im Folgenden Beispiele genant werden. Die in den Beispielen aufgeführten Copolymere können statistische, alternierende, Block-, Graft-Copolymere oder Kombinationen derselben sein. Die in den Beispielen genannten Verhältnisse m : n beziehen sich auf die relativen molaren Anteile der Co-Einheiten und stellen keine absoluten Größenangaben für die Polymerkettenlängen dar. Die Anzahl der Wiederholungseinheiten pro Polymerkette liegt bei den Beispielen über 5, bevorzugt über 10, besonders bevorzugt über 20.
(1) Homo- und Copolymere der allgemeinen Struktur 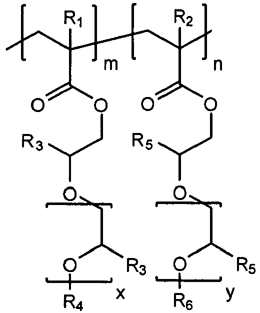 mit R1, R2 = -H, -Alkyl, bevorzugt -H und -CH3, besonders bevorzugt -CH3,
   mit R3, R5 = -H, -Alkyl, Alkenyl, Alkinyl, Aryl, bevorzugt -H und -CH3, besonders bevorzugt -H, in jedem Fall muß gelten: wenn R3, ≠ -H, dann R5 = -H,
   mit R4, R6 = -H, -Alkyl, Alkenyl, Alkinyl, Aryl, bevorzugt -H und / oder -CH3, besonders bevorzugt -CH3.
   Wenn R3, R5 = -H und R4, R6 = -CH3, x = 1 und y = 7,5, dann liegt m : n bevorzugt zwischen 95 : 5 und 90 : 10, besonders bevorzugt bei 93 : 7. Wenn R3, R5 = -H und R4, R6 = -CH3, x = 1 und y = 4,5, dann liegt m : n bevorzugt zwischen 93 : 7 und 80 : 20, und besonders bevorzugt bei 85 : 15.
(2) Homo- und Copolymere der allgemeinen Struktur 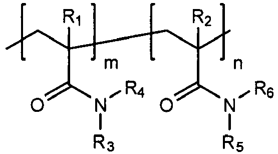 mit R1, R2 = -H, -Alkyl, bevorzugt -H und -CH3, besonders bevorzugt -H,
   mit R3, R4, R5 und R6 = -H, -Alkyl, Alkenyl, Alkinyl, Aryl, bevorzugt -R3 = -Isopropyl, -R4 = -H, -R5 und -R6 = -C2H5, - CH3 oder -H, besonders bevorzugt -R3 = -Isopropyl und -R4 = - H und m : n = 100 : 0,
   und / oder R3, R4, R5 und 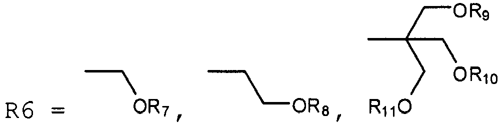 mit R7, bis R11 = -H, -Alkyl, Alkenyl, Alkinyl, Aryl, - Alkyloyl, mindestens ein R = H.
(3) Homo- und Copolymere der allgemeinen Struktur 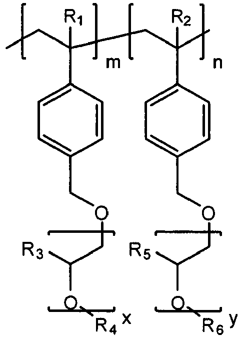 mit R1, R2 = -H, -Alkyl, bevorzugt -H und -CH3, besonders bevorzugt -H, mit R3, R5 = -H, -Alkyl, Alkenyl, Alkinyl, Aryl, bevorzugt -H und -CH3, besonders bevorzugt -H (in jedem Fall gilt: wenn R3, ≠ -H, dann R5 = -H), mit R4, R6 = -H, -Alkyl, Alkenyl, Alkinyl, Aryl, bevorzugt -H und / oder -CH3, besonders bevorzugt -H.
   Wenn R1, R2 = -H, und R4, R6 = -H, x =3 und y = 4, dann liegt m : n bevorzugt zwischen 65 : 35 und 45 : 55, besonders bevorzugt zwischen 60 : 40 und 50 : 50.
(4) Homo- und Copolymere der allgemeinen Struktur 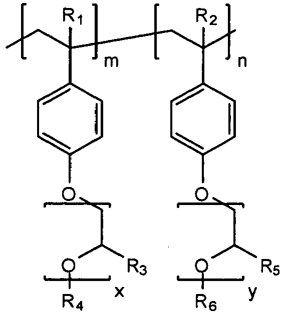 mit R1, R2 = -H, -Alkyl, bevorzugt -H und -CH3, besonders bevorzugt -H, mit R3, R5 = -H, -Alkyl, Alkenyl, Alkinyl, Aryl, bevorzugt -H und -CH3, besonders bevorzugt -H (in jedem Fall gilt: wenn R3, ≠ -H, dann R5 = -H), mit R4, R6 = -H, -Alkyl, Alkenyl, Alkinyl, Aryl, bevorzugt -H und / oder -CH3, besonders bevorzugt -H.
(5) Homo- und Copolymere der allgemeinen Struktur 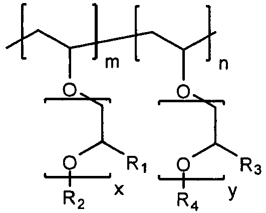 mit R1, R3 = -H, -Alkyl, Alkenyl, Alkinyl, Aryl, bevorzugt -H und -CH3, besonders bevorzugt -H (in jedem Fall: wenn R3 ≠ - H, dann R5 = -H), mit R2, R4 = -H, -Alkyl, Alkenyl, Alkinyl, Aryl, bevorzugt -H und / oder -CH3, besonders bevorzugt -CH3.
(6) Homo- und Copolymere der allgemeinen Struktur 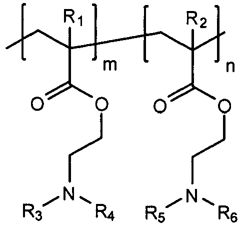 mit R₁, R₂ = -H, -Alkyl, bevorzugt -H
   R₃, R₄, R₅, R₆ = -Alkyl, Alkenyl, Alkinyl, Aryl, bevorzugt -Alkyl, besonders bevorzugt R₃, R₄ = -CH₃, R₅, R₆ = -C₂H₅, m : n zwischen 0 : 100 und 10 : 90.
(7) Homo- und Copolymere der allgemeinen Struktur 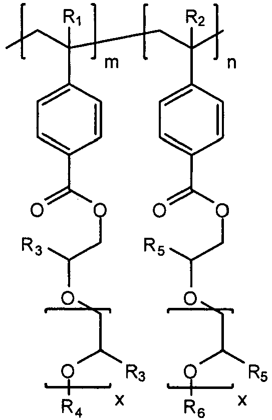 mit R1, R2 = -H, -Alkyl, bevorzugt -H und -CH3, besonders bevorzugt -H, mit R3, R5 = -H, -Alkyl, Alkenyl, Alkinyl, Aryl, bevorzugt -H und -CH3, besonders bevorzugt -H (in jedem Fall gilt: wenn R3, ≠ -H, dann R5 = -H), mit R4, R6 = -H, -Alkyl, Alkenyl, Alkinyl, Aryl, bevorzugt -H und / oder -CH3, besonders bevorzugt -H.
(8) Homo- und Copolymere der allgemeinen Struktur 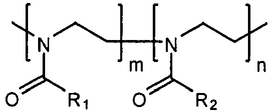 mit R1, R2 = -H, -Alkyl, Alkenyl, Alkinyl, Aryl.
(9) Homo- und Copolymere der allgemeinen Struktur 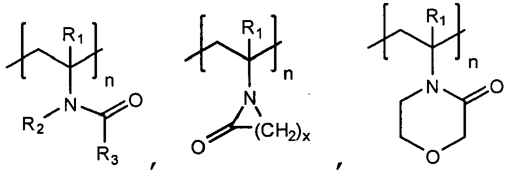 oder Copolymere der drei Elemente in frei wählbaren Zusammensetzungen
   mit R1 = -H, -Alkyl, bevorzugt -H und -CH3, besonders bevorzugt -H, mit R2, R3 = -H, -Alkyl, Alkenyl, Alkinyl, Aryl, mit 2 ≤ x ≤ 10, bevorzugt 3 ≤ x ≤ 6.
(10) Copolymere der allgemeinen Struktur 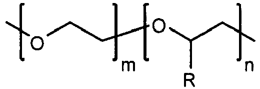 mit -R = -H, -Alkyl, Alkenyl, Alkinyl, Aryl, bevorzugt -R = - Alkyl, besonders bevorzugt -R = -CH3.
(11) Homo- und Copolymere der allgemeinen Struktur 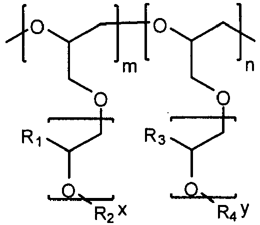 mit R1, R3 = -H, -Alkyl, Alkenyl, Alkinyl, Aryl, bevorzugt -H und -CH3 (in jedem Fall gilt: wenn R1, ≠ -H, dann R3 = -H), mit R2, R4 = -H, -Alkyl, Alkenyl, Alkinyl, Aryl, bevorzugt -H und / oder -CH3.
(12) Homo- und Copolymere der allgemeinen Struktur 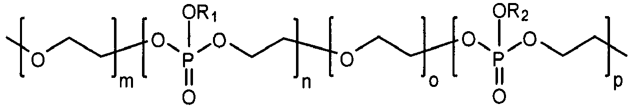 mit R1, R2 = -H, -Alkyl, Alkenyl, Alkinyl, Aryl.
(13) Homo- und Copolymere der allgemeinen Struktur 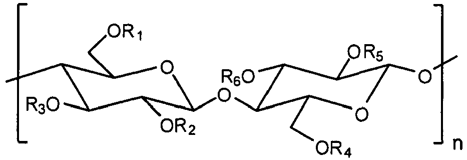 mit R1 bis R6 = -H, -Alkyl, Alkenyl, Alkinyl, Aryl, - Alkyloyl, mindestens zwei R = H.
(14) Homo- und Copolymere analoger Struktur wie unter 13, basierend auf anderen Polysaccharidgerüsten als Cellulose
(15) Homo- oder Copolymere mit elastin-ähnlichen Einheiten
(16) Copolymere aller oben genannten Einheiten
(17) Copolymere aller oben genannten Einheiten mit anderen Monomeren

Das thermoresponsive Polymermaterial 13 kann durch Physisorption, elektrostatische Wechselwirkung, molekulare Verbindung oder durch kovalente Bindung an der Trägerflächen gebunden sein. Das thermoresponsive Polymermaterial 13 kann zusätzlich funktionelle Gruppen tragen, die die Bindung an die Oberfläche erleichtern oder erst ermöglichen. Je nach Oberfläche und Immobilisierungsmethode kann es sich dabei z. B. um hydrophobe Gruppen, Thiole, Disulfide, Silane, Amine, Carboxylgruppen, Azide oder Maleiimide handeln.

Die Kultivierungseinrichtung 30 umfasst das schematisch gezeigte Kultivierungsgefäß 31 mit einem Boden 32 und einer umlaufenden Seitenwand 33. Das Kultivierungsgefäß 31 ist zur Aufnahme eines flüssigen Kultivierungsmediums 34 vorgesehen, das durch eine Fluidikeinrichtung umfassend eine Zufuhrleitung 35 und eine Auslassleitung 36 in das Kultivierungsgefäß 31 eingeführt bzw. entfernt werden kann. Das erfindungsgemäße Substrat 10 bildet den Boden 32 des Kultivierungsgefäßes 31. Alternativ kann das Substrat 10 ein auf einen Boden des Kultivierungsgefäßes 31 gelegtes Teil sein.

Die Kultivierungseinrichtung 30 ist mit einem Thermostaten (nicht dargestellt), mit dem eine Temperatur T₁ des Kultivierungsmediums 34 einstellbar ist, und mit einer Pumpeinrichtung (nicht dargestellt) ausgestattet, mit der das Kultivierungsmediums 34 durch das Kultivierungsgefäß 31 gefördert werden kann. Der Raum über der Trägerfläche 11 des Substrats 10 ist mit dem temperierbaren Kultivierungsmedium 34 bedeckt. Durch eine Beaufschlagung des Kultivierungsgefäßes 31 mit einer Druckdifferenz kann eine Strömung des Kultivierungsmediums 34 durch das Kultivierungsgefäß 31 erzeugt werden.

Des Weiteren kann die Kultivierungseinrichtung 30 mit einer Beobachtungseinrichtung, z.B. einem Mikroskop, einer Messeinrichtung, z.B. einem Temperatursensor (nicht dargestellt) und/oder einer Manipulatoreinrichtung ausgestattet sein, z.B. eine zusätzliche Zufuhrleitung umfasst, durch die eine Zellsuspension in das Kultivierungsgefäß 31 gespült werden kann.

Zellen 21, 22 verschiedener Zelltypen, z. B. dermale Fibroblasten, Keratinozyten und primäre Melanozyten, werden wie folgt gezielt angeordnet. In einem ersten Verfahrensschritt wird eine Suspension der Zellen 21 des ersten Zelltyps, z. B. dermale Fibroblasten mit dem Kultivierungsmedium 34 in das Kultivierungsgefäß 31 geleitet. Das Kultivierungsmedium 34 hat eine Temperatur T₁ (z. B. 25°C) unterhalb der Schalttemperatur (z. B. 33°C) des thermoresponsiven Polymermaterials 13. Durch eine Aktivierung der Temperierungselemente 41, 42 wird eine Temperaturverteilung der Trägerfläche 12 derart gebildet, dass in bestimmten Segmenten die Temperatur des thermoresponsiven Polymermaterials 13 oberhalb der Schalttemperatur liegt. In diesem Fall wird in den interessierenden Segmenten mit Heizelementen geheizt. In diesen Segmenten (z. B. 15 in Figur 1B) (Adhäsions-Segmente) mit einer Temperatur T₂ oberhalb der Schalttemperatur können die Zellen 21 anhaften, während in anderen Segmenten mit einer Temperatur unterhalb der Schalttemperatur keine Adhäsionsfähigkeit gegeben ist. Im Ergebnis sind ausschließlich in den Adhäsions-Segmenten die Zellen des ersten Zelltyps 21 adhärent angeordnet, wie in Figur 1A schematisch gezeigt ist.

In einem weiteren Schritt wird eine Suspension des zweiten Zelltyps 22, z. B. Keratinozyten mit dem Kultivierungsmedium 34 in das Kultivierungsgefäß 31 geleitet. Die Temperierungselemente 41, 42 werden so geschaltet, dass in den bisher leer gebliebenen Segmenten die Schalttemperatur des thermoresponsiven Polymermaterials 13 überschritten wird. Im dargestellten Beispiel werden die Heizelemente eingeschaltet. Im Ergebnis können die Zellen 22 des zweiten Zelltyps auf den bisher freien Segmenten anhaften. Auf den bereits mit Zellen 21 vom ersten Zelltyp besetzten Segmenten können Zellen, die lediglich Monolagen bilden, nicht oder nur langsam anhaften. Werden zu einem geeigneten Zeitpunkt (z.B. nach 3 bis 12 Stunden) die überständigen Zellen 22 des zweiten Zelltyps entfernt, bleiben Inseln aus haftenden Zellen 22 des zweiten Zelltyps inmitten des strukturierten Rasens aus Zellen 21 des ersten Zelltyps, während diese selbst nicht besiedelt sind. Alternativ zum Einschalten aller Heizelemente kann das gesamte Substrat auf eine Temperatur von z. B. 37 °C gebracht werden.

Im Ergebnis wird gezielt eine Anordnung der Zellen 21, 22 der verschiedenen Zelltypen realisiert, wie schematisch in Figur 1B gezeigt ist. Die Umgebung z. B. der Zellen 21 des ersten Zelltyps kann gezielt z. B. durch Zellen 22 des zweiten Zelltyps gebildet werden, wie es für die *in* vitro-Kultivierung, insbesondere die Gewebeinduktion oder die Nachbildung von Stammzellnischen von Vorteil ist.

In den Figuren 1A und 1B ist eine Variante der Erfindung illustriert, bei der die Temperierungselemente die Heizelemente 41 umfassen. Da die Temperatur T₁ kleiner als die Schalttemperatur des thermoresponsiven Polymermaterials 3 ist, kann das Anhaften der Zellen allein durch ein Einschalten der gewünschten Heizelemente 41 induziert werden. Zusätzlich umfassen die Temperierungselemente die Kühlelemente 42. Des Weiteren kann das Kultivierungsmedium 34 eine Temperatur T₁ oberhalb der Schalttemperatur des thermoresponsiven Polymermaterials 13 aufweisen und eine Aktivierung der Kühlelemente zur Absenkung der Temperatur unterhalb der Schalttemperatur in den jeweils gewünschten Segmenten vorgesehen sein.

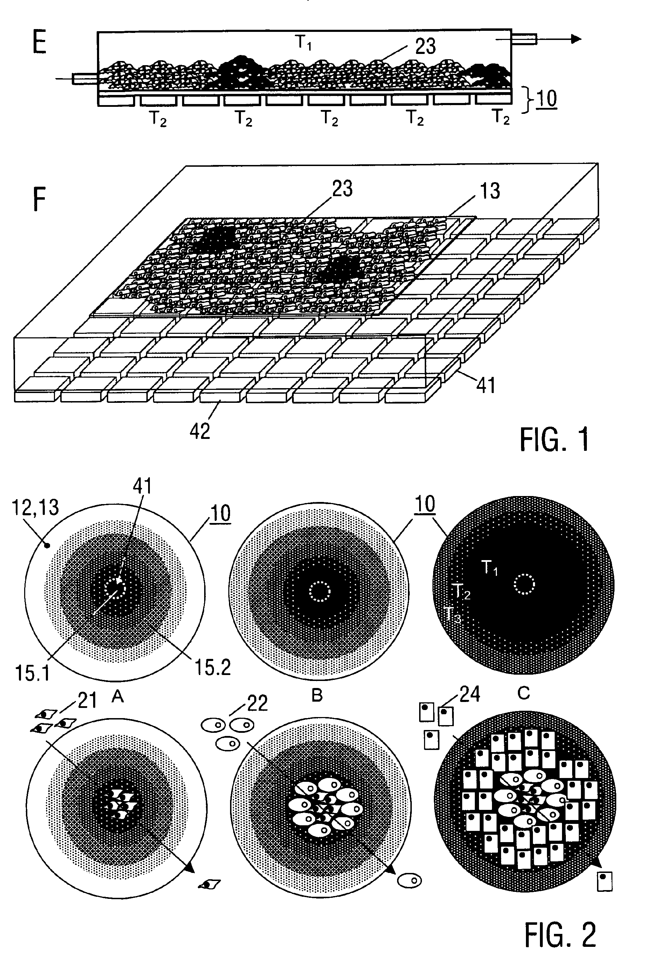

Die Figuren 1C bis 1F zeigen weitere Schritte der Kultivierung der Zellen 21, 22 auf dem Substrat 10. Durch das Wachstum der Zellen entstehen in den zugehörigen Segmenten zunächst Zellgruppen 21.1, 22.1 (Figuren 1C, 1D), die beim weiteren Wachstum zu einem dreidimensionalen Zellverband 23 zusammenwachsen (Figuren 1E, 1F). Im weiteren Verfahren kann der Zellverband 23 nach Abkühlung aller Temperierungselemente 41, 42 unterhalb der Schalttemperatur des thermoresponsiven Polymermaterials 13 vom Substrat 10 abgelöst und einer weiteren Bearbeitung unterzogen werden.

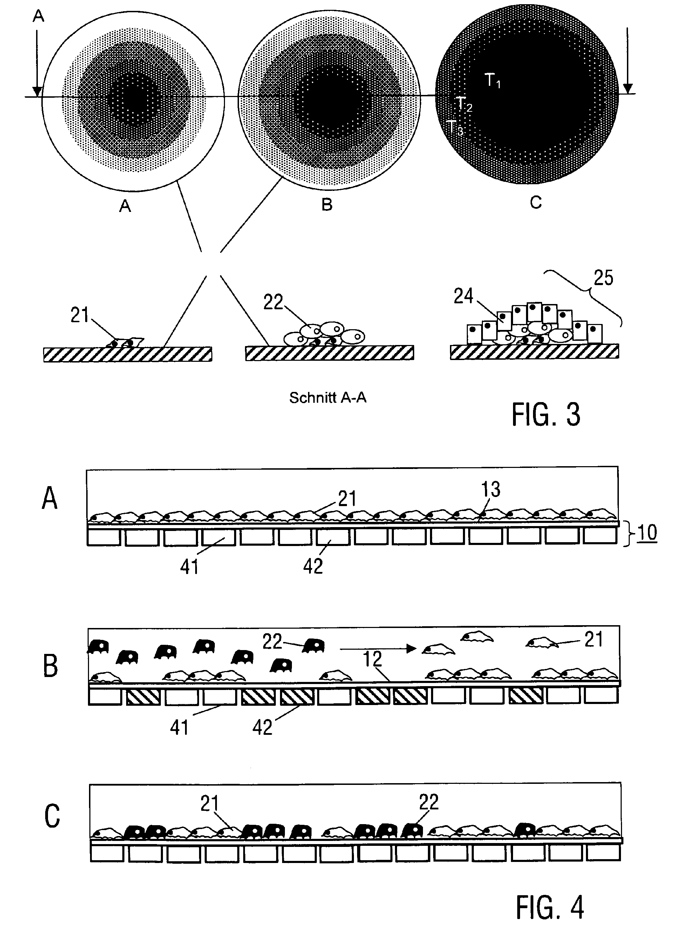

Die Figuren 2 und 3 zeigen ein Substrat 10 (keine Ausführungsform der Erfindung) mit einem einzigen Temperierungselement 41. Erfindungsgemäß kann das Substrat 10 mit einer konzentrischen Anordnung ringförmiger Temperierungselemente (nicht dargestellt) ausgestattet sein. Das in Draufsicht gezeigte Substrat 10 hat z. B. eine kreisrunde Trägerfläche 12, die mit thermoresponsivem Polymermaterial 13 beschichtet ist. Das Temperierungselement 41 (gepunktet gezeichnet) befindet sich unterhalb des Substrats 10 in der Mitte der Trägerfläche 12. Bei Betätigung des Temperierungselements 41 (Heizung oder Kühlung) bildet sich ein konzentrisches Temperaturfeld auf der Trägerfläche 12. Mit der Aktivierung des Temperierungselements 41 wird nicht nur die Temperatur des zentralen, kreisförmigen Segments 15.1, sondern auch die Temperatur der Segmente (z.B. 15.2) eingestellt, welche das zentrale Segment 15.1 konzentrisch umgeben. Das Substrat 10 befindet sich in einer Kultivierungseinrichtung (nicht dargestellt), die z.B. wie in Figur 1 schematisch gezeigt aufgebaut ist.

Mit dem in den Figuren 2 und 3 gezeigten Substrat 10 können verschiedene Zelltypen durch die lokale Kontrolle der Oberflächentemperatur aufeinander folgend in den konzentrischen Segmenten angeordnet werden. So zeigt Figur 2 eine zeitliche Reihenfolge (A, B, C), die z. B. Minuten bis Stunden oder auch Tage umfasst. Die obere Reihe von Figur 2 zeigt die Bildung des Temperaturfeldes, während die untere Reihe die verschiedenen Phasen der Anordnung biologischer Zellen illustriert.

Zum Zeitpunkt A werden Zellen 21 eines ersten Zelltyps eingespült. Die Temperatur des Substrats liegt zunächst unterhalb der Polymerschalttemperatur. In der Mitte des Feldes wird geheizt, so dass ein nach außen abfallender Temperaturgradient entsteht. Nur im schwarz gezeigten zentralen Segment 15.1 liegt die Temperatur der Oberfläche über der Schalttemperatur des Polymermaterials 13. Dadurch können die Zellen 21 ausschließlich im zentralen Segment 15.1 anhaften. Werden überschüssige Zellen 21 aus dem Kultivierungsraum herausgespült (Pfeil unten in A) und Zellen 22 eines weiteren Zelltyps eingespült und der Bereich der höheren Temperatur erweitert (B), so haften die neuen Zellen 22 rings um die erste Zellgruppe am Polymermaterial. Die Schritte des Einspülens und kontrollierten Anhaftens werden mit Zellen 24 eines dritten Zelltyps wiederholt (C), wodurch ringförmige Zellanordnungen entstehen.

Das Beispiel in Figur 3 zeigt ein Verfahren analog zu dem der Figur 2, wobei hier drei Zelltypen (21, 22, 24) verwendet werden, die aufeinander anhaften und wachsen können, z. B. vorbestimmte Stammzell-Typen und für diese geeignete Feeder-Zellen (z. B. mitoseinhibierte embryonale Fibroblasten). Wie im Schnitt A - A illustriert ist, entstehen dreidimensionale Zellaggregate 25 (Gewebekörper), wie sie für die Gewebeinduktion oder Modellgewebe für pharmakologische Untersuchungen benötigt werden. Wie unter Bezug auf Figur 2 beschrieben, wird die mit dem thermoresponsiven Polymermaterial 13 beschichtete Trägerfläche 12 mit den verschiedenen Zelltypen nacheinander durch lokale Kontrolle der Oberflächentemperatur besiedelt.

Der sich in Abhängigkeit von der Zeit vergrößernde Bereich der Trägerfläche mit einer Temperatur oberhalb der Schalttemperatur des Polymermaterials kann durch zunehmende Temperaturerhöhung am einzigen Temperierungselement 41 oder alternativ durch ein schrittweises Einschalten von konzentrisch angeordneten ringförmigen Temperierungselementen erreicht werden. Es ist möglich, dass andere als die gezeigten Gradienten und Temperaturverteilungen erzeugt werden und damit frei wählbare Zellmuster und Zellanordnungen Schritt für Schritt aufgebaut werden können.

Figur 4 illustriert den Ablauf der Ansiedlung verschiedener Zelltypen ausgehend von einer Monolage von Zellen 21 eines ersten Zelltyps, die auf dem Substrat 10 mit den Temperierungselementen 41, 42 gebildet ist. Analog zu dem unter Bezug auf Figur 1 beschriebenen Verfahren sollen Zellen planar strukturiert auf der Trägerfläche 12 angesiedelt werden. Im Gegensatz zu Figur 1 werden zuerst die Zellen 21 des ersten Zelltyps als Monolage aufgebracht und anschließend in strukturierter Form abgelöst, um den Zellen 22 des zweiten Zelltyps Platz zu schaffen.

Gemäß Figur 4A ist die Temperatur der Trägerfläche 12 so eingestellt, dass das Polymermaterial 13 das Anhaften der Zellen erlaubt. Alle Temperierungselemente 41, 42 sind gleichartig aktiv oder abgeschaltet. Beispielsweise ist, wenn die Temperatur des Kultivierungsmediums unterhalb der Schalttemperatur des Polymermaterials 13 liegt, eine Heizung der gesamten Trägerfläche 12 auf eine Temperatur oberhalb der Schalttemperatur vorgesehen. Wenn die Temperatur des Kultivierungsmediums oberhalb der Schalttemperatur des Polymermaterials 13 liegt und auch die Temperatur der Trägerfläche 12 bestimmt, ist eine Heizung nicht erforderlich.

Gemäß Figur 4B ist gezeigt, dass z.B. durch Kühlen der Trägerfläche 12 mittels der Temperierungselemente 42 die Zellen 21 sich ablösen und ausgeschwemmt werden können. Anschließend wird an diesen Segmenten die Temperatur wieder erhöht, und Zellen 22 eines zweiten Zelltyps werden eingespült. Diese siedeln sich auf den freien Segmenten an, wie es in Figur 4C gezeigt ist. Dieses Verfahren kann mit wechselnden Adhäsionsmustern und wechselnden Zelltypen wiederholt werden, bis eine Anordnung adhärenter Zellen gebildet ist, bei der für jede Zelle eine gezielt eingestellte Mikroumgebung gegeben ist.

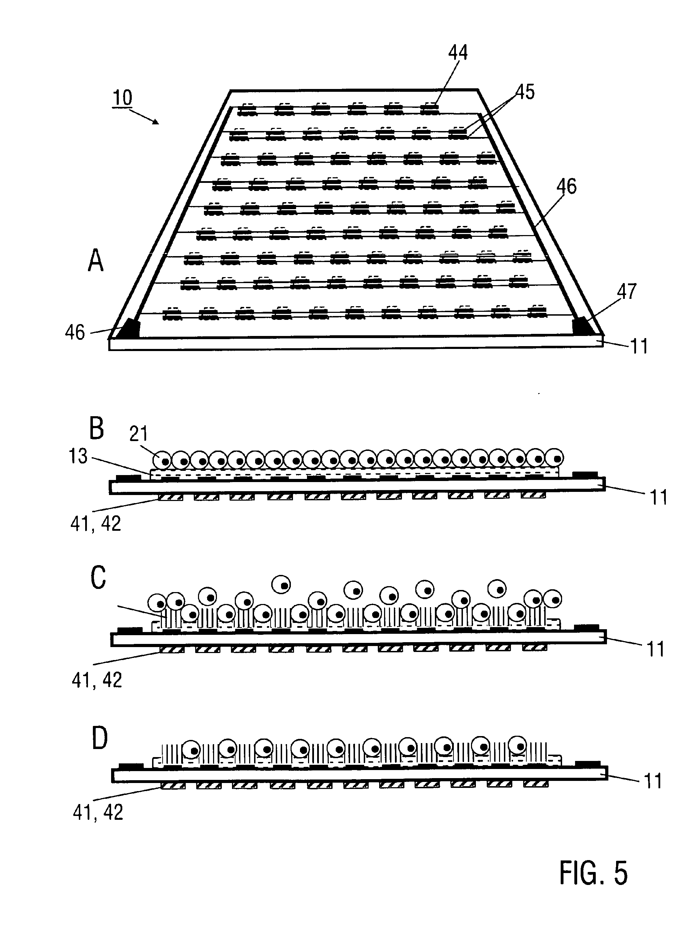

Figur 5 zeigt eine Erweiterung des in Figur 1 gezeigten Substrats 10, bei dem eine thermische Strukturierung nicht nur mit den Temperierungselementen 41, 42 auf der Unterseite des Substratkörpers 11, sondern auch durch auf der Substratoberfläche angebrachte Temperierungselemente 44, 45 (Heiz- oder Kühlelemente) erreicht wird. Die oberen Temperierungselemente 44, 45 ermöglichen vorteilhafterweise eine Feinabstimmung der Temperatur der Substratoberfläche. Die Temperierungselemente 45, 45 sind über elektrische Verbindungen 46 mit Kontakten 47 auf dem Substratkörper 11 verbunden (siehe Perspektivansicht in Figur 5A). Jedes Temperierungselement 44, 45 umfasst ein Elektrodenpaar mit einem Heizwiderstand oder einem Peltierelement, das über die elektrischen Verbindungen 46 mit einer Betriebsspannung beaufschlagt werden kann. Die elektrischen Verbindungen 46 sind so geschaltet, dass die Temperierungselemente 44, 45 in jeweils kleinen Gruppen (hier z. B. Liniengruppen) angesteuert werden können. Alternativ könnten individuell ansteuerbare Temperierungselemente 44, 45 vorgesehen sein.

Über den Temperierungselementen 44, 45 liegt deckend das thermoresponsive Polymermaterial, auf dem bei einer Temperatur oberhalb der Schalttemperatur die Zellen 21 anhaften und wachsen (siehe Schnittansicht in Figur 5B). Unter dem Substratkörper 11 befinden sich Temperierungselemente 41, 42, mit denen die Temperatur lokal einstellbar ist.

Das Substrat 10 gemäß Figur 5 ermöglicht analog zu der in Figur 1 gezeigten Ausführungsform die Herstellung einer definierten Zellanordnung. Wird über den Temperierungselementen 43, 44 die Temperatur erniedrigt, so lösen sich die Zellen an diesen Stellen ab (Figur 5C). Figur 5D zeigt, wie sich dann eine geordnete Zellanordnung ergibt. Durch Erwärmen können die freien Stellen wieder anhaftfähig für Zellen gemacht werden.

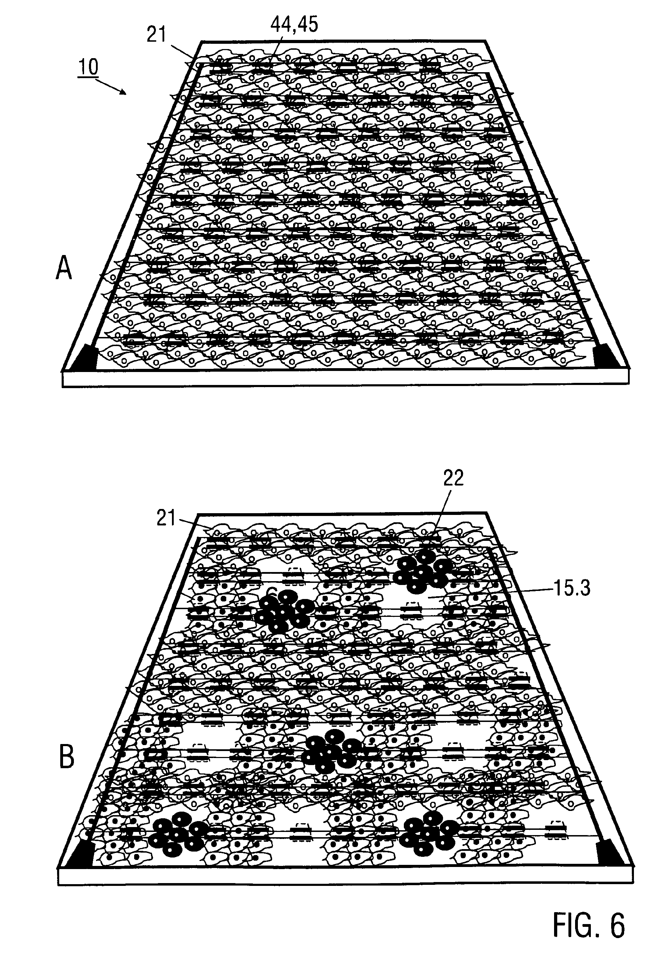

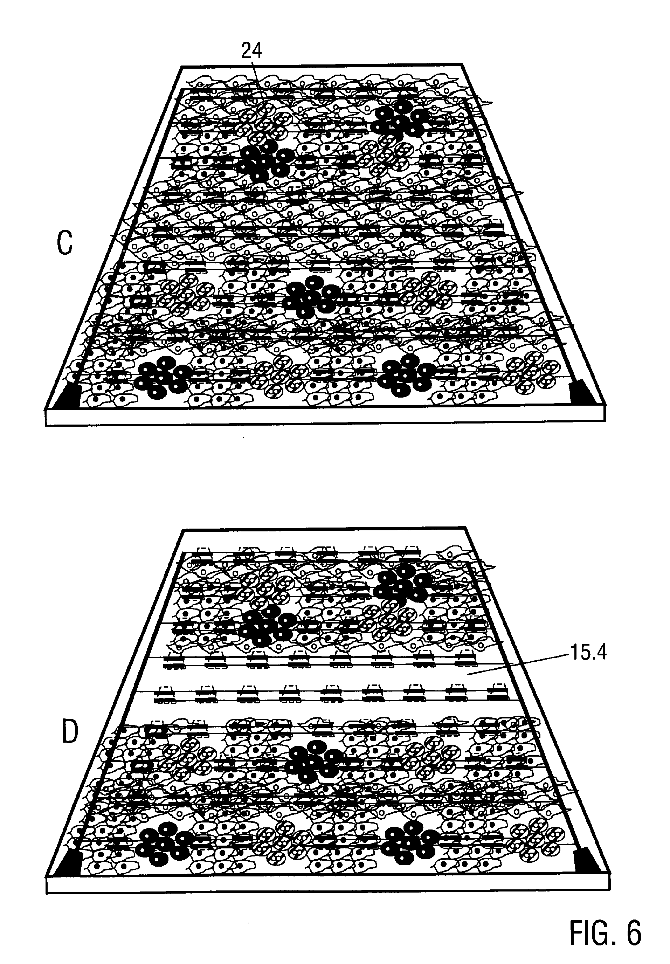

Figur 6 zeigt in der Folge A bis D den Aufbau von geordneten Zellgruppen auf dem Substrat 10 gemäß Figur 5. In Figur 6A wachsen Zellen 21 zu einer konfluenten Zellschicht auf dem thermoresponsiven Polymermaterial, das die Temperierungselemente 44, 45 bedeckt. Figur 6B zeigt, wie durch Abkühlen bestimmter Segmente eine lokal begrenzte Ablösung von Zellen erreicht wird, um Leer-Segmente 15.3 zu schaffen, und anschließend durch eine Erwärmung die betreffenden Segmente mit Zellen 22 neu besiedelt werden.

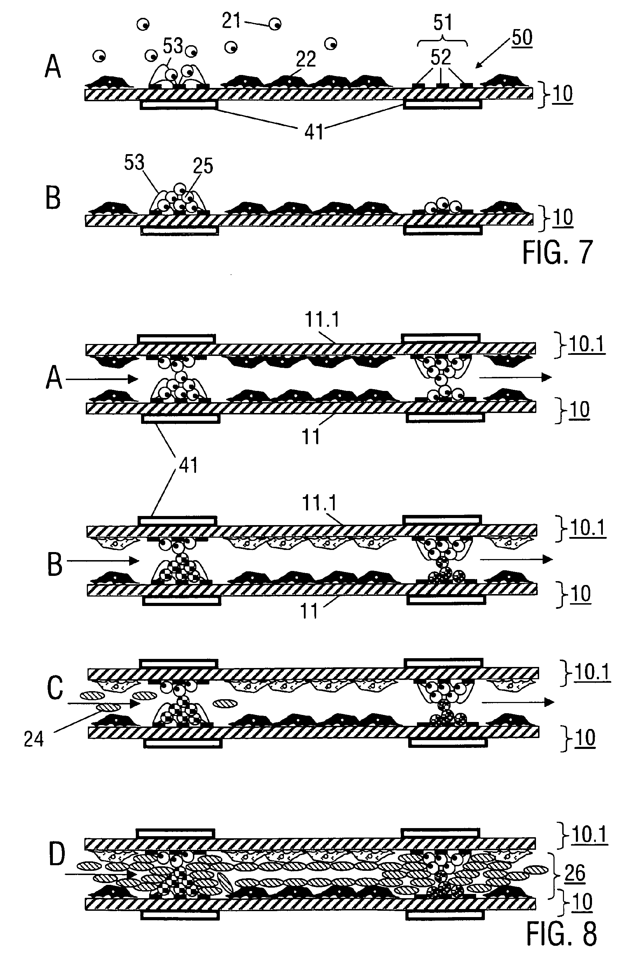

Figur 6C zeigt das Einfügen von Zellen 24 eines weiteren Zelltyps an einem zuvor freigemachten Segment. Die oberen Temperierungselemente 44, 45 können, wie in Figur 6C beispielhaft gezeigt und unter Bezug auf Figur 7 weiter beschrieben wird, auch zur Erzeugung dielektrophoretischer Kräfte verwendet werden. Hierzu werden durch Anlegen einer hochfrequenten Wechselspannung an die Elektrodenpaare an den freien Segmenten Zellen aus der Suspension über elektrische Polarisation (positive Dielektrophorese) angezogen, so dass sie exakt auf den freien Segmenten angelagert werden und danach wachsen. Der Vorteil einer solchen Anordnung ist, dass auch Zellen, die aufeinander anhaften und wachsen können, separat anordenbar werden. Figur 6D verdeutlicht das Freiwerden breiterer Segment-Streifen 15.4 durch Abkühlung des gewünschten Bereichs der Substrat-Oberfläche und Ablösen der Zellen durch Auflösen der Polymerstruktur.

Die strukturierte Zellanordnung mit Hilfe dielektrophoretischer Kräfte ist auch in Figur 7 illustriert. Bei dieser Ausführungsform der Erfindung sind auf der Unterseite des Substratkörpers 11 des erfindungsgemäßen Substrats 10 Temperierungselemente 41, z. B. Heizelemente vorgesehen (die Kühlelemente des erfindungsgemäßen Substrats 10 sind nicht gezeigt). Auf der Oberseite des Substratkörpers 11 ist eine Elektrodeneinrichtung 50 vorgesehen, die Elektrodenelemente 51 umfasst. Die Elektrodenelemente 51 sind über elektrische Verbindungen mit einer Spannungsquelle verbunden, die zur Erzeugung einer hochfrequenten Wechselspannung eingerichtet ist (nicht dargestellt). Der Aufbau der Elektrodenelemente 51 und der elektrischen Verbindungen auf der Oberseite des Substrats 10 kann vorgesehen sein, wie es z.B. in den Figuren 5 und 6 gezeigt ist. Jedes der Elektrodenelemente 51 umfasst eine Gruppe von Elektrodenstreifen 52, mit denen hochfrequente elektrische Felder 53 über dem Substratkörper 11 erzeugt werden können. Es ist jeweils ein Elektrodenelement 51 einem der Temperierungselemente 41 zugeordnet. Die Elektrodenelemente 51 sind den Temperierungselementen 41 gegenüberliegend angeordnet, so dass an den Segmenten, in denen mit den Temperierungselementen 41 die Temperatur lokal einstellbar ist, die maximale Wirkung der hochfrequenten elektrischen Felder 53 erzielt wird.

In den Figuren 7A und 7B ist gezeigt, wie Zellen 21 als Suspension mit dem Kultivierungsmedium eingespült werden und dann mit den hochfrequenten Feldern 53, die über den Elektrodenelementen 51 erzeugt werden, zu den Elektrodenelementen 51 gezogen werden (positive Dielektrophorese). Die Sammlung von Zellen an den Adhäsions-Segmenten mittels positiver Dielektrophorese hat den Vorteil, dass die anfängliche Besiedlung durch Adhäsion auf dem Substrat 10 verstärkt wird, um dreidimensionale Zellaggregate 25 zu formen.

Gemäß Figur 8 können zwei Substrate 10, 10.1, z. B. wie sie in Figur 7 gezeigt sind, mit einem geringen Abstand, z. B. 50 µm bis 2 mm, einander gegenübergestellt werden, um einen mikrofluidischen Kultivierungsraum (Kammer oder Kanal) zu bilden. Der Abstand wird mit einem Spacer (nicht dargestellt) zwischen den Substratkörpern 11, 11.1 eingestellt. Auf diese Weise werden weitere Möglichkeiten der dreidimensionalen Gestaltung komplexer Zellaggregate geschaffen.

Beispielsweise werden gemäß Figur 8A Zellen auf beiden Seiten des Kultivierungsraums angesiedelt. Ein Kultivierungsmedium (Nährlösung) durchströmt den Kultivierungsraum, so dass sich gewebeähnliche Zellaggregate bilden. Teile der Zellaggregate können analog zu den oben beschriebenen Verfahren durch Schalten der Temperierungselemente 41 und eine damit induzierte Temperaturänderung abgelöst und ausgetauscht werden. So können z. B. gemäß Figur 8B auf den Substraten 10, 10.1 Zellen oder Zellaggregate verschiedener Zelltypen angeordnet werden. Im Ergebnis kann die Zahl der Zellkombinationen erhöht werden. Gemäß Figur 8C wird in den Kultivierungsraum nach Besiedelung gemäß Figur 8B eine weitere Suspension mit weiteren Zellen 24 eingespült, die den Zwischenraum komplett oder teilweise ausfüllen oder durchwachsen (Figur 8D). Dadurch kann eine Zellschicht 26 generiert werden. Die Dichte der Zellschicht 26 kann eingestellt werden, wie es z.B. für künstliches Gewebe, Futtermittel oder synthetische Lebensmittel erwünscht ist. Eine Trennung der Zellschicht 26 von den Substraten 10, 10.1 kann durch eine Abkühlung der SubstratOberflächen unterhalb der Schalttemperatur und eine Entfernung der Substrate 10, 10.1 erreicht werden.

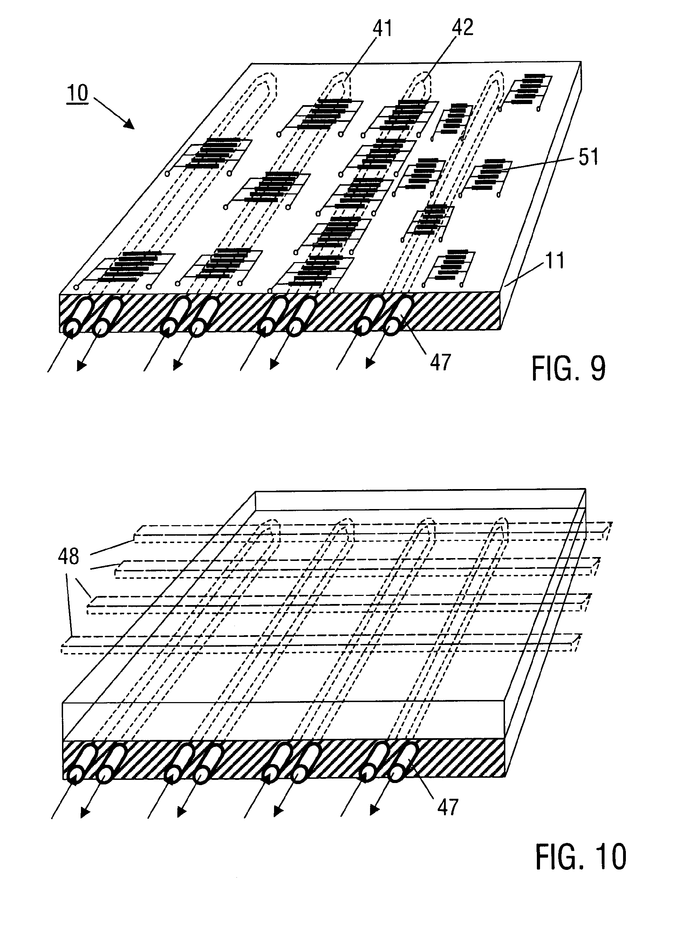

Figur 9 zeigt ein Substrat 10, bei dem die Temperierungselemente Konvektionsheizelemente 41 oder Konvektionskühlelemente 42 umfassen (in der dargestellten Form keine Ausführungsform der Erfindung). Im Substratkörper 11 sind Fluidleitungen 47 (Kanäle oder Durchflüsse) enthalten, durch die eine Kühl- oder Heizflüssigkeit oder ein Gas strömen kann. Die Geometrie (Größe, Form, Position) der Fluidleitungen 47 ist in Abhängigkeit von der Geometrie der einzustellenden Segmente des Substrats gewählt. Abweichend von der in Figur 9 beispielhaft gezeigten geraden Streifengeometrie können zum Beispiel gekrümmte Leitungen vorgesehen sein. Mit der Streifengeometrie kann eine linienartige Ablösung oder Adhäsion von Zellen erzielt werden. Für eine zusätzliche Strukturierung sind auf der Oberseite des Substratkörpers 11 elektrisch betätigbare Temperierungselemente (Widerstandselemente oder Peltierelemente, Ausführungsform der Erfindung, nicht dargestellt) und/oder Elektrodenelemente 51, z. B. mit Interdigitalelektroden, zur lokalen Abstoßung oder Sammlung von Zellen vorgesehen.

Weitere Freiheitsgrade bei der Einstellung der Temperaturverteilung der Trägerfläche des Substrats können erzielt werden, wenn Fluidleitungen 47, 48 in verschiedenen Tiefen im Substratkörper 11 mit verschiedenen Richtungen verlaufen (Figur 10, keine Ausführungsform der Erfindung). Die Fluidleitungen 47, 48 verlaufen z. B. rechtwinklig zueinander. Eine lokal abgegrenzte Temperatureinstellung wird in Segmenten ermöglicht, die sich über Überschneidungspunkten der Fluidleitungen 47, 48 befinden.

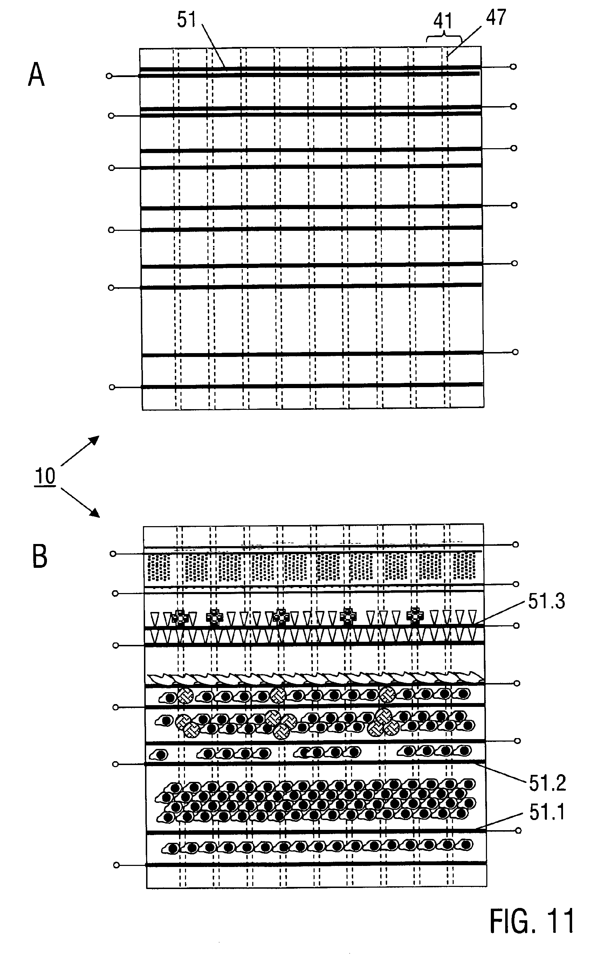

Ein weiteres Substrat 10 mit einer Kombination aus Konvektionsheizelementen 41 (und/oder Konvektionskühlelementen) mit Elektrodenelementen 51 ist in Figur 11 gezeigt (keine Ausführungsform der Erfindung). Figur 11A illustriert den Aufbau des Substrats 10, in dessen Substratkörper die Fluidleitungen 47 und rechtwinklig zu diesen streifenförmige Elektrodenelemente 51 verlaufen. Die Elektrodenelemente 51 umfassen jeweils ein Elektrodenpaar, das dazu vorgesehen ist, mittels positiver oder negativer Dielektrophorese Zellen und/oder Moleküle zu sammeln oder abzustoßen. Der Abstand der Elektroden eines Elektrodenpaares kann z. B. im Bereich von 5 µm bis 500 µm gewählt sein. Figur 11B veranschaulicht beispielhaft, dass durch eine Temperatureinstellung Zellen abgelöst oder adhäriert und zusätzlich mit den Elektrodenelementen Zellen oder Moleküle gesammelt oder abgestoßen werden können. Falls Zellen oder Moleküle von den elektrischen Feldern abgestoßen werden, können sie sich im Feldminimum zwischen den Elektrodenelementen (z. B. 51.1, 51.2) sammeln. Andernfalls kann eine Sammlung auf den Elektrodenelementen (z. B. 51.3) vorgesehen sein.

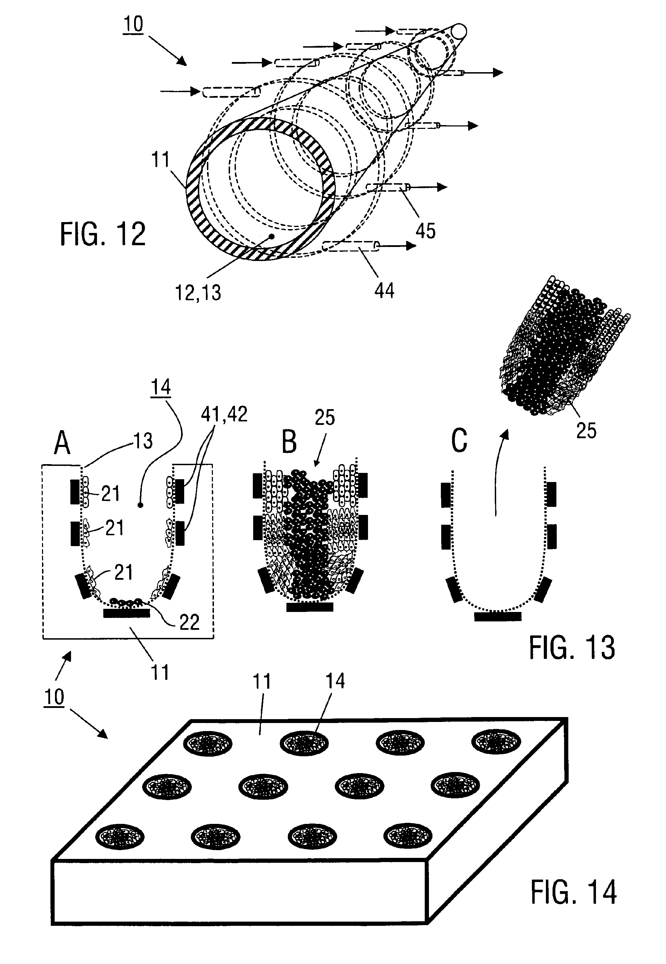

Die Figuren 12 bis 14 zeigen Ausführungsformen der Erfindung, bei denen gekrümmte Trägerflächen 12 vorgesehen sind. So umfasst gemäß Figur 12 der Substratkörper 11 des Substrats 10 eine Kapillare, deren Innenseite die Trägerfläche 12 mit dem thermoresponsiven Polymermaterial 13 bildet. Die Kapillare besteht z. B. aus Glas oder einem Kunststoff. Sie hat eine Länge zum Beispiel im Bereich von 10 mm bis 200 mm und einen Innendurchmesser zum Beispiel im Bereich von 100 µm bis 10 mm. Die Temperierungselemente 44, 45 umfassen ringförmige Kühl- oder Heizelemente auf einer Außenseite der Kapillare. Es sind z. B. Konvektionsheizelemente oder Konvektionskühlelemente (keine Ausführungsform der Erfindung) vorgesehen, die um die Kapillare gewickelt und mit einer Flüssigkeit oder einem Gas durchströmt (siehe Pfeile) und dadurch temperiert werden. Die Temperierungselemente 44, 45 sind in axialer Richtung der Kapillare verteilt angeordnet. Gemäß der Erfindung werden elektrisch betätigbare Temperierungselemente, z.B. unter Verwendung von Widerstandsdraht, verwendet.

Mit der Ausführungsform gemäß Figur 12 erfolgt eine gezielte Ablage von Zellen verschiedener Zelltypen im Inneren der Kapillare, die einen Kultivierungsraum bildet. Ein Kultivierungsmedium mit suspendierten Zellen wird durch die Kapillare geleitet. Die Temperierungselemente 44, 45 werden so angesteuert, dass vorbestimmte Ringsegmente der inneren Trägerfläche 12 eine Adhäsion von Zellen fördern, während an den übrigen Ringsegmenten eine Adhäsion unterbunden wird. Nach einer gezielten Anordnung von Zellen können diese in der Kapillare einer weiteren Kultivierung unterzogen werden, bis die Kapillare mit den Zellen dicht gefüllt ist. Anschließend kann das Zellmaterial aus der Kapillare entfernt werden, zum Beispiel indem die Temperierungselemente 44, 45 eine Temperatur unterhalb der Schalttemperatur des Polymermaterials 13 einstellen. Im gekühlten Zustand kann das Zellmaterial aus der Kapillare geschoben werden. Gemäß einer Variante der Erfindung kann die Kapillare Sollbruchstellen aufweisen. Dies ermöglicht vorteilhafterweise, dass die Kapillare zur vereinfachten Entfernung der Zellaggregate in Teilstücke zerteilt werden kann.

Figur 13 zeigt eine weitere Ausführungsform des erfindungsgemäßen Substrats 10 (teilweise dargestellt) mit einer gekrümmten Trägerfläche 12, die mindestens einen Napf 14 im Substratkörper 11 bildet. Diese Ausführungsform dient dem sequenziellen Aufbau von Zellanordnungen aus verschiedenen Zelltypen im Napf 14. Vorteile ergeben sich insbesondere für die Nachbildung von Mikroumgebungen in Vertiefungen (Nischen) natürlichen Gewebes, in denen sich 3-dimensionale Zellstrukturen bilden. Beispiele natürlicher Mikroumgebungen im Form von Kavitäten oder Nischen sind Haarfollikeln, StammzellNischen oder Strukturen in Lebergewebe, wie die Glisson-Trias mit polarisierten Hepatozyten.

Auf der Trägerfläche 12 ist das thermoresponsive Polymermaterial 13 (gepunktet illustriert) angeordnet. Die Temperierungselemente 41, 42 sind in den Substratkörper 11 eingebettet oder ebenfalls auf der Trägerfläche 11 und vom Polymermaterial 13 bedeckt angeordnet. Die Temperierungselemente 41, 42 umfassen in Abhängigkeit von der Gestaltung des Substrats 10 und der gewünschten Verfahrensweise der Anordnung der Zellen Heiz- und Kühlelemente. Die Temperierungselemente 41, 42 können z. B. ringförmig den Napf 14 umgebend in axialer Richtung verteilt oder in azimuthaler Richtung mit einzelnen Elementen strukturiert sein. Der Substratkörper 11 besteht aus einem porösen Material, wie z. B. Keramik, Glas oder Kunststoff. Die Porösität des Substratkörpers 11 ist so gewählt, dass dieser für flüssiges Kultivierungsmedium (Nährmedium) durchlässig und für biologische Zellen undurchlässig ist.

Der Napf 14 hat z. B. die folgenden Dimensionen: Durchmesser: 20 µm bis 2 mm, Tiefe: 20 µm bis 5 mm, Abstand zum benachbarten Napf: 500 µm bis 10 mm. So können innerhalb eines Substratkörpers 11 eine Vielzahl von Näpfen 14 angeordnet sein, wie schematisch in Figur 14 illustriert ist.

Die gezielte Anordnung biologischer Zellen in einem Napf 14 gemäß den Figuren 13 oder 14 erfolgt mit den folgenden Schritten. Zunächst werden die Temperierungselemente 41, 42 so angesteuert, dass Zellen 21 von einem ersten Zelltyp in den gewünschten Segmenten der Trägerfläche 12 lokal angesiedelt werden. Angrenzende Segmente bleiben frei (Figur 13A). Anschließend können in einem weiteren Verfahrensschritt in frei gebliebenen Segmenten Zellen 22 von einem weiteren Zelltyp deponiert werden. Mit diesem Verfahren können sequenziell auch mehr als zwei verschiedene Zelltypen auf der Trägerfläche 12 angeordnet werden. Im Ergebnis ist der Innenwand des Napfes 14 mit einer Zellschicht ausgekleidet, die an der seitlichen Wand des Napfes 14 z. B. interfollikuläre Fibroblasten oder interfollikuläre Keratinozyten und am Boden des Napfes 14 z. B. epitheliale Stammzellen eines Haarfollikels umfassen.

Nach der Ablage von Zellen verschiedener Zelltypen auf der Trägerfläche 12 erfolgt eine Kultivierung, die insbesondere ein Wachstum und/oder eine Differenzierung der Zellen im Napf 14 umfasst. Im Ergebnis bildet sich im Napf 14 ein dichtes Zellaggregat 25, wie z. B. ein Gewebepropf (Figur 13B). Durch die simultane Abkühlung aller Temperierungselemente 41, 42 auf eine Temperatur unterhalb der Schalttemperatur des Polymermaterials 13 kann die Adhäsion des Zellaggregats 25 vermindert und dieses aus dem Napf 14 gezogen werden (Figur 13C). Anschließend kann eine weitere Kultivierung oder eine Kryokonservierung des Zellaggregats 25 vorgesehen sein.

Die Array-Anordnung von Näpfen 14 gemäß Figur 14 eignet sich insbesondere für Testzwecke. So können auf einem gemeinsamen Substrat 10 in den verschiedenen Näpfen 14 Zellen verschiedener Zelltypen kombiniert werden, um verschiedene Gewebetypen zu erhalten. Die Versorgung mit einem Nähemedium kann über ein das Substrat 10 überdeckendes Kultivierungsmedium, bei Verwendung eines porösen Substratkörpers 11 durch den Substratkörper 11 und/oder durch Versorgungsleitungen im Substrat (nicht dargestellt) erfolgen.

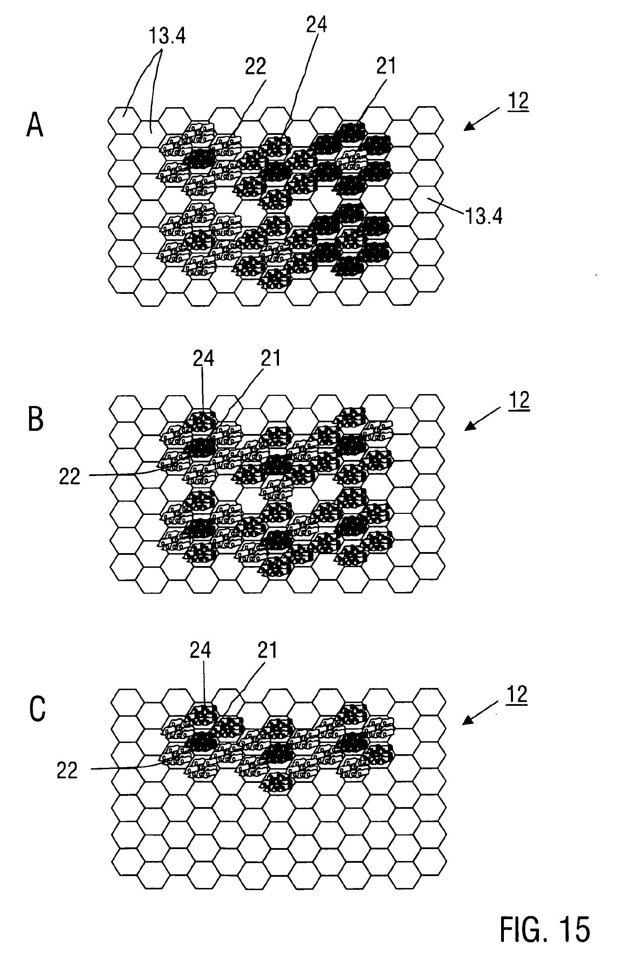

Figur 15 illustriert schematisch die kombinatorische Herstellung von Zellaggregaten aus Zellen verschiedener Zelltypen. Dabei werden Zellen miteinander kombiniert, um das Verhalten von Zellen in einer Mikroumgebung in Abhängigkeit von den umgebenden Zellen, z. B. von den Signalfaktoren der umgebenden Zellen zu untersuchen. Die kombinatorische Herstellung von Zellaggregaten umfasst einen Austausch umgebender Zellen gemäß allen interessierenden Permutationen. Die hierfür erforderliche Anordnung von Zellen verschiedenen Zelltypen wird vorzugsweise mit dem erfindungsgemäßen Verfahren realisiert.

Die Trägerfläche 12 des erfindungsgemäßen Substrats ist in schematisch illustrierte hexagonale Segmente 13.4 unterteilt, denen jeweils ein Temperierungselement (nicht dargestellt) zugeordnet ist. Die Temperatur der einzelnen Segmente 13.4 ist einzeln und unabhängig von der Temperatur der umgebenden Segmente steuerbar. Auf die Segmente 13.4 werden sequenziell Zellen 21, 22, 24 verschiedener Zelltypen aufgebracht. Aus Übersichtlichkeitsgründen wird hier nur auf drei Zelltypen Bezug genommen. Die kombinatorische Herstellung von Zellaggregaten ist entsprechend aber auch mit mehr Zelltypen möglich.

Durch die Steuerung der lokalen Zelladhäsion in Abhängigkeit von der Temperatur der Segmente 13.4 kann jede Kombination von Nachbarschaften von Zellen verschiedener Zelltypen permutativ realisiert werden. Zellen können in verschiedenen Zellumgebungen kultiviert und untersucht werden. Im Ergebnis liefert das Verfahren für jeden Zelltyp Informationen über die Adhäsionseigenschaften und Kultivierungseigenschaften, wie z. B. das Wachstum und die Differenzierung in Abhängigkeit von den umgebenden Zelltypen.

Gemäß der Figur 15A ist vorgesehen, dass Zellen von jedem Zelltyp jeweils von Zellen eines anderen Zelltyps gleichmäßig umschlossen sind. So kann jede Kombination getestet werden, welcher Zelltyp mit welchen Nachbarn die besten Kultivierungsergebnisse erzielt. Im Figur 15B ist illustriert, wie die Abhängigkeit der Zellen 21 eines ersten Zelltyps von dem Mengenverhältnis von Zellen 22, 24 weiterer Zelltypen in unmittelbarer Nachbarschaft getestet werden kann. Mit dem erfindungsgemäßen Verfahren können die Mengenverhältnisse durch die gezielte Anordnung der Zellen 22, 24 in der Umgebung der Zellen 21 variiert werden. Des Weiteren kann mit der kombinatorischen Herstellung von Zellaggregaten untersucht werden, wie die Zellen 21 des ersten Zelltyps bei konstantem Mengenverhältnis der Zellen 22, 24 weiterer Zelltypen von der geometrischen Anordnung der Zellen 22, 24 abhängen (Figur 15C). Die verschiedenen Anordnungen können mit dem erfindungsgemäßen Verfahren durch die gezielte Deposition von Zellen auf den umgebenden Segmenten in der Nachbarschaft der Zellen 21 eingestellt werden.

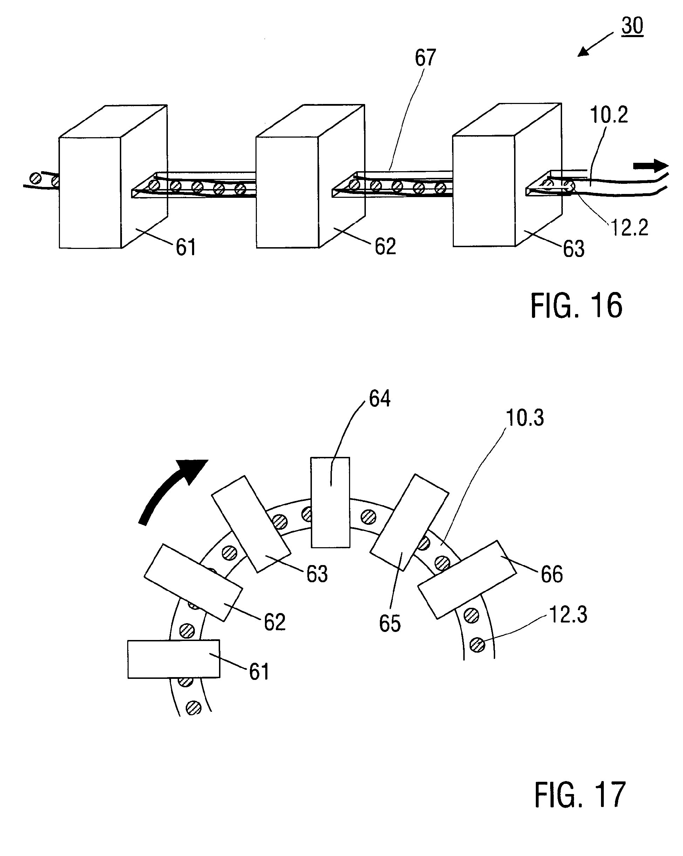

Die kombinatorische Herstellung von Zellaggregaten gemäß Figur 15 kann von der illustrierten Variation in Zellschichten (Monolagen) zu einer Variation in der dritten Dimension erweitert werden. In Abhängigkeit von der Zahl verschiedener Zelltypen und Zahl verschiedener Parameter, die getestet werden sollen, kann sich ein kombinatorisches Verfahren von erheblicher Komplexität ergeben. In diesem Fall ist eine Automatisierung des Verfahrens mit einer Kultivierungseinrichtung von Vorteil, die mehrere Kultivierungsstationen umfasst, wie im folgenden unter Bezug auf die Figuren 16 und 17 beispielhaft beschrieben ist.

Eine Automatisierung des erfindungsgemäßen Verfahrens ist insbesondere von Vorteil, wenn die sequenzielle Besiedelung lokal definierter Segmente auf dem Substrat eine genaue Kontrolle der Substrattemperatur aller Segmente erfordert und/oder Zellen einer größeren Anzahl von Zelltypen (z. B. mehr als drei Zelltypen) gezielt angeordnet werden. Für die Automatisierung hat die Kultivierungseinrichtung 30 vorzugsweise einen modularen Aufbau mit einer Vielzahl von Kultivierungsstationen 61, 62, 63 ....

Gemäß Figur 16 sind beispielhaft drei Kultivierungsstationen 61, 62, 63 gezeigt, die ortsfest angeordnet und für die Einstellung von Kultivierungsbedingungen an einem beweglichen Substrat 10.2 eingerichtet sind. Das Substrat 10.2 umfasst z. B. einen flexiblen Streifen, der durch die Kultivierungsstationen 61, 62, 63 und zwischen diesen durch einen Kanal 67 mit einem Kultivierungsmedium bewegt werden kann. Auf der Oberfläche oder im Substratmaterial des Substrats 10.2 befinden sich Trägerflächen 12.2, die jeweils mit thermoresponsivem Polymermaterial bedeckt und für eine temperaturabhängige Anhaftung biologischer Zellen ausgelegt sind. Zwischen den Trägerflächen 12.2 ist die Oberfläche des Substrats 10.2 so gebildet, dass dort unabhängig von der Temperatur keine Zellen anhaften können.

Jede Kultivierungsstation 61, 62, 63, ... ist eine Einheit zur Durchführung eines Verfahrensschrittes des erfindungsgemäßen Kultivierungsverfahrens und gegebenenfalls weiterer Verfahrensschritte, wie z. B. einer Nährstoffzufuhr oder einer Kultivierung. Da die Kultivierungseinrichtung für eine Vielzahl von Schritten einsetzbar sein muss, gibt es für jeden Schritt eines gewünschten Verfahrens eine Kultivierungsstation, die modular in die Kultivierungseinrichtung 30 eingesetzt werden kann. In Abhängigkeit von der konkreten Anwendung werden genau die Kultivierungsstationen aktiviert, die für das gewünschte Verfahren notwendig sind, während andere, unnötige Kultivierungsstationen inaktiv sind und/oder aus der Kultivierungseinrichtung 30 entnommen werden.

Die Bewegung des Substrats 10. 2 und der Betrieb der Kultivierungsstationen 61, 62, 63, ... werden mit einer Steuereinrichtung (nicht dargestellt) gesteuert. Die Steuereinrichtung setzt das gewünschte Verfahren in ein passendes Protokoll zur Einstellung der Geschwindigkeit des Substrats 10.2 und/oder der Temperaturen von Temperierungselementen an den einzelnen Kultivierungsstationen um.

Da auf den Trägerflächen 12.2 lebende Zellen wachsen, wird das gesamte Substrat 10.2 permanent durch ein Kultivierungsmedium geführt, das durch den geschlossenen Kanal 67 und auch an den Kultivierungsstationen 61, 62, 63, ... die Trägerfläche 12.2 bedeckt.

Die Temperierungselemente des erfindungsgemäßen Substrats können in das Substrat 10.2 integriert sein und mit diesem bewegt werden. In diesem Fall sind an den Kultivierungsstationen 61, 62, 63, .... Berührungskontakte zur Beaufschlagung der Temperierungselemente mit Betriebsströmen, z. B. für Heiz- und/oder Kühlzwecke vorgesehen. Alternativ können die Kultivierungsstationen 61, 62, 63, .... jeweils mit Temperierungselementen ausgestattet sein, die mit dem Substrat 10.2 in thermischen Kontakt gebracht werden.

Das Substrat 10.2 umfasst z. B. ein homogenes Band, wie z. B. einen Kunststoffstreifen, der vorzugsweise optisch transparentem Material hergestellt ist. In diesem Fall ist das Substrat 10.2 z. B. aus einem der Kunststoffe PC (Polycarbonat), COP (cyclisches Olefin-Copolymer, Topas), TCPS (Zellkultur-Polystyren), PP (Polypropylen) oder PE (Polyethylen) hergestellt. Die Bildung der Trägerflächen 12.2 kann durch eine topografische und/oder eine chemische Strukturierung der Oberfläche des Kunststoffstreifens erfolgen. Zusätzlich können Markierungen, wie z. B. Farbmarken angebracht sein, um die Position und gegebenenfalls weitere Informationen über die Trägerflächen 12.2 auszulesen.

Die Oberfläche des Substrats 10.2 kann vollständig oder teilweise funktionalisiert sein, wie z. B. durch eine Metallfilm-Beschichtung, eine Ionenplasma-Behandlung, eine Funktionalisierung mit Silanverbindungen, wie z. B. APTES (Aminopropyl-Triethoxy-Silan), oder DACH (1,2-Diamino-cyclohexan), eine Immobilisierung von Proteinen, Nukleinsäuren und Polymeren, insbesondere Biopolymeren. Des Weiteren sind die Trägerflächen 12.2 mit thermoresponsiven Polymeren zur Steuerung der Zelladhäsion bedeckt.

Alternativ kann das Substrat 10.2 ein heterogenes Band, insbesondere ein Trägerband mit Einsätzen umfassen, welche die Trägerflächen 12.2 bilden. Im Trägerband können z. B. Aussparungen zur Aufnahme von Schalen vorgesehen sein, im denen Segmente für die Besiedelung mit Zellen angeordnet sind. In diesem Fall ist der Aufbau der Substrats 10.2 zwar komplizierter. Vorteilhafterweise können die Einsätze wie Schalen, z. B. wie Petrischalen, gebildet sein, so dass das Kultivierungsmedium ausschließlich an den Einsätzen vorhanden ist. In diesem Fall kann auf den Kanal 67 zur Führung eines Kultivierungsmediums in der Umgebung des Substrats 10.2 verzichtet werden.

Figur 17 illustriert eine abgewandelte Variante einer Kultivierungseinrichtung 30, bei der die Kultivierungsstationen 61 bis 66 kreisförmig eingeordnet sind. In diesem Fall kann das Substrat 10.2 einen Ring, z. B. aus Metall oder Kunststoff, umfassen. Das Substrat 10.3 kann als homogener Ringstreifen mit den auf der Substratoberfläche gebildeten Trägerflächen 12.3 oder mit den Einsätzen gebildet sein, wie dies oben beschrieben ist. Durch Drehung des ringförmigen Substrats 10.3 werden die einzelnen Trägerflächen 12.3 durch die Kultivierungsstationen 61 bis 66 geleitet, so dass in diesen die jeweiligen Trägerflächen 12.3 einzeln prozessiert werden können.

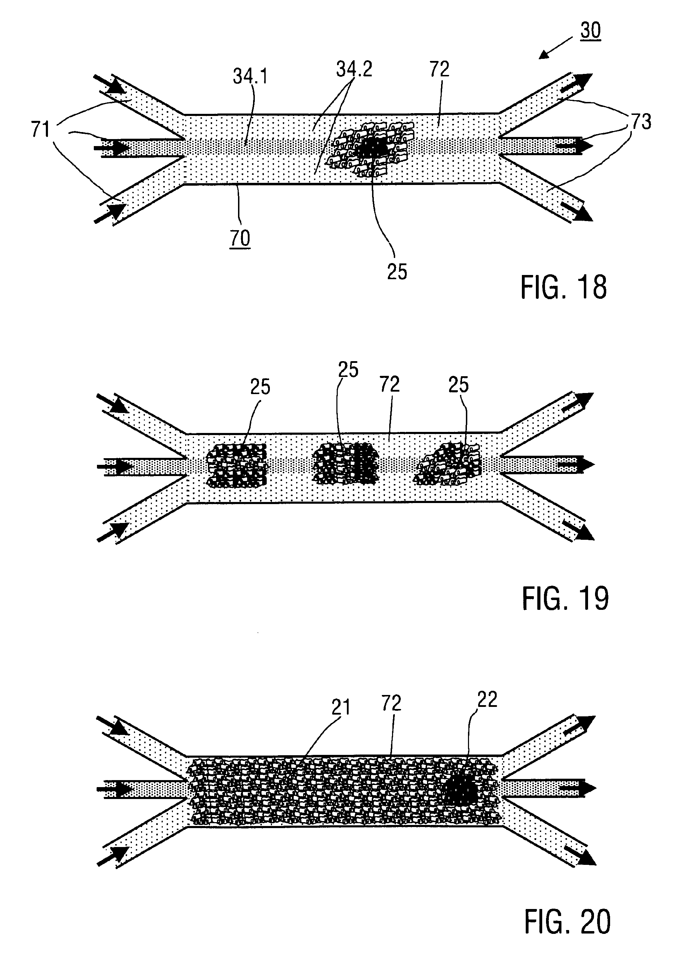

Die Figuren 18 bis 21 zeigen Varianten der Erfindung, bei denen die Kultivierungseinrichtung 30 ein fluidisches Mikrosystem 70 umfasst. Das erfindungsgemäße Substrat 10 ist in das fluidische Mikrosystem 70 integriert. Vorteilhafterweise wird damit die Steuerung der Mikroumgebung optimiert, da ein kontinuierlicher Medienfluss durch das Mikrosystem 70 über die Zellen eine gleichmäßigere Zusammensetzung der Bestandteile des Kultivierungsmediums gewährleistet, als dies bei einem zyklischen Wechsel des Kultivierungsmediums mit einer zyklischen Abreicherung von Nährstoffen und Anreichung von Stoffwechselprodukten im Kultivierungsmedium der Fall ist. Ein weiterer Vorteil der Verwendung fluidischer Mikrosysteme 70 besteht in der Möglichkeit, den Flüssigkeitsstrom des Kultivierungsmediums in verschiedenen Leitungen des Mikrosystems 70 zu teilen, so dass mindestens zwei verschieden zusammengesetzte Kultivierungsmedien den Zellen zugeführt werden. Es lassen sich Konzentrationsgradienten und/oder eine polare Umgebung für die Zellen bilden, wie dies bei statischen Systemen nicht erreichbar wäre.

Allgemein umfasst das fluidische Mikrosystem 70 Fluidkanäle 71, 73, mindestens eine Fluidkammer 72, die mit den Fluidkanälen 71, 73 verbunden ist, und Fluidikelemente (nicht dargestellt, wie z. B. Pumpen, Ventile oder Dielektrophorese-Elektroden) zur Manipulation des Kultivierungsmediums oder der in diesem enthaltenen Bestandteile. Das erfindungsgemäße Substrat 10 ist Teil der Fluidkammer 72. Beispielsweise wird der Boden der Fluidkammer 72 durch das Substrat 10 gebildet. Entsprechend sind die oben beschriebenen Temperierungselemente in den Boden der Fluidkammer 72 integriert. Das fluidische Mikrosystem 70 kann hergestellt werden, wie dies an sich aus der Herstellung fluidischer Mikrosysteme für die dielektrophoretische Manipulation biologischer Zellen bekannt ist.

Figur 18 illustriert ein Beispiel eines fluidischen Mikrosystems 70, bei dem mit der Fluidkammer 72 drei Eingangs-Fluidkanäle 71 und drei Ausgangs-Fluidkanäle 73 verbunden sind, die mit Hilfe externer Pumpen (nicht dargestellt) mit verschiedenen Kultivierungsmedien durchströmt werden. Ein erstes Kultivierungsmedium 34.1 fließt in der Mitte der Fluidkammer 72, während ein zweites Kultivierungsmedium 34.2 an das erste Kultivierungsmedium 34.1 angrenzend einen Hüllstrom bildet. In der Fluidkammer 72 wird mit dem erfindungsgemäßen Verfahren sequenziell ein Zellaggregat 25 aufgebaut, das durch den strukturierten Medienstrom aus den ersten und zweiten Kultivierungsmedien 34.1, 34.2 überströmt wird. Entsprechend werden für die Zellen gezielt Kultivierungsbedingungen, wie z. B. eine fluidisch polare Umgebung bereitgestellt.

Um den Einfluss der fluidischen Umgebung auf Zellaggregate 25 zu untersuchen, werden gemäß Figur 19 in der Fluidkammer 72 mehrere Zellaggregate 25 mit verschiedenen räumlichen Anordnungen der Zellen gebildet. So kann beispielsweise eine Anordnung von Zellen verschiedener Zelltypen getestet werden, die parallel zum strukturierten Medienstrom, hierzu senkrecht oder unregelmäßig verteilt angeordnet sind.

Figur 20 illustriert ein Verfahren, bei dem der Einfluss parakriner Faktoren auf Zellfunktionen untersucht wird. Zunächst wird auf dem gesamten Boden der Fluidkammer 72 eine geschlossene Lage der Zellen 21 eines ersten Zelltyps gebildet (siehe Figur 6). Anschließend wird durch eine lokale Temperaturerniedrigung ein kleiner Bereich der Zellschicht in der Nähe der Ausgangs-Fluidkanäle 73 durch Zellen 22 eines anderen Zelltyps ersetzt. Das Kultivierungsmedium, das nun die Zellen 22 erreicht, wurde durch den Weg über die Zellen 21 mit parakrinen Faktoren angereichert, so dass die Zellen 22 kontinuierlich mit frisch konditioniertem Medium versorgt werden.

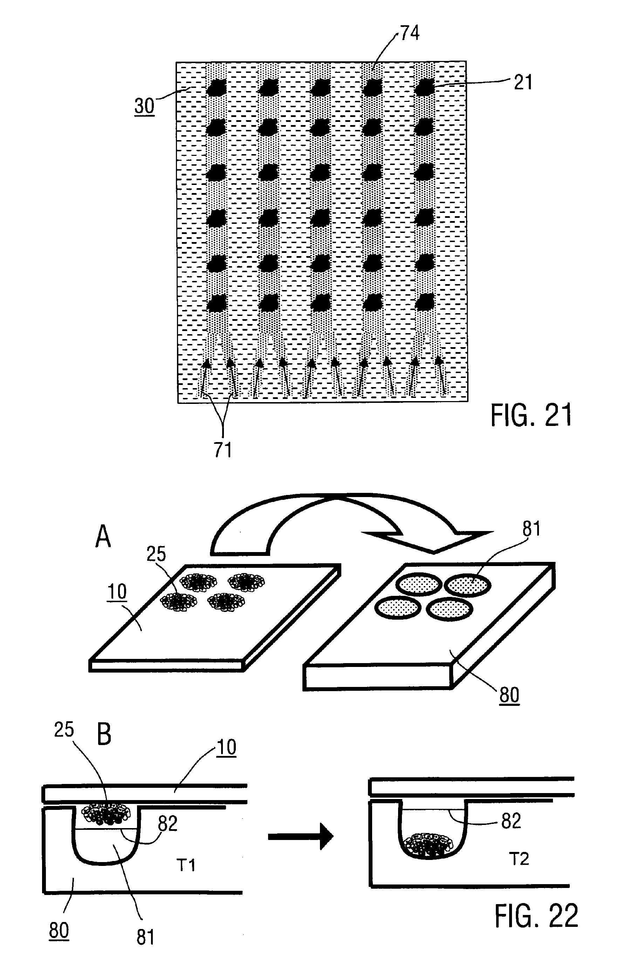

Mit einem fluidischen Mikrosystem 70 können auch Zellaggregate aus verschiedenen Zelltypen kombinatorisch hergestellt werden, wie oben unter Bezug auf Figur 15 beschrieben wurde. Das fluidische Mikrosystem 70 enthält, wie in Figur 21 gezeigt ist, ein Array von Fluidkammern 74, auf deren Boden jeweils ein erfindungsgemäßes Substrat gebildet und thermisch strukturiert ist. Durch die Eingangs-Fluidkanäle 71 werden Kultivierungsmedien verschiedener Zusammensetzung eingebracht, die laminar parallel durch die Fluidkammern 74 fließen und so Konzentrationsgradienten über den Zellen 21 bilden.

Erfindungsgemäß kann nach der gezielten Anordnung von Zellen verschiedener Zelltypen eine Ablösung eines Zellaggregats von dem Substrat und eine Kryokonservierung des Aggregats vorgesehen sein, wie beispielhaft in den Figuren 22 bis 23 illustriert ist. Gemäß Figur 22A können Zellaggregate 25 von einem erfindungsgemäßen Substrat 10 auf ein Konservierungssubstrat 80 überführt werden. Das Konservierungssubstrat 80 enthält Näpfe 81 mit einer Größe und Anordnung entsprechend der Größe und Anordnung der Zellaggregate 25 auf dem Substrat 10. In den Näpfen 81 ist ein Einfriermedium für die Kryokonservierung angeordnet, das z. B. ein Kryoprotektivum enthalten kann.

Die Zellaggregate 25 werden z. B. durch ein Auflegen des Substrats 10 auf das Konservierungssubstrat 80 und ein Eindrücken der Zellaggregate 25 in die Näpfe 81 übertragen. Die Situation mit dem aufgelegten Substrat 10 auf dem Konservierungssubstrat 80 ist beispielhaft mit einem einzelnen Napf 81 in Figur 22B gezeigt. Das Zellaggregat 25, das zunächst an dem Substrat 10 haftet, wird durch eine Absenkung der Substrattemperatur unterhalb der Schalttemperatur des thermoresponsiven Polymermaterials freigegeben, so dass es unter der Wirkung der Gravitation auf den Boden des Napfes 81 fällt. Die Freigabe des Zellaggregats 25 vom Substrat 10 kann durch eine mechanische Schwingung z. B. durch einen PiezoKristall unterstützt werden. Im Napf 81 erfolgt die Abkühlung des Zellaggregats 25 im Einfriermedium 82 mit an sich bekannten Methoden der Kryokonservierung, z. B. einer kontrollierten Abkühlungsrate von Grad pro Minute bis zu einer Temperatur unterhalb von - 70°C.

Die in der vorstehenden Beschreibung, den Zeichnungen und den Ansprüchen offenbarte Merkmale der Erfindung können sowohl Einzelnen als auch in Kombination für die Verwirklichung der Erfindung in ihren verschiedenen Ausgestaltungen von Bedeutung sein.

## Patentansprüche

1. Substrat (10, 10.1, 10.2, 10.3), das zur Aufnahme biologischer Zellen (20, 21, 22, 23, 24, 25) im adhärenten Zustand eingerichtet ist, umfassend:
- einen Substratkörper (11, 11.1) mit einer Trägerfläche (12), auf der ein thermoresponsives Polymermaterial (13) angeordnet ist, und
- eine Vielzahl von Widerstands-Heizelementen (41, 44), die mit dem Substratkörper (11, 11.1) fest verbunden sind, wobei
- mit jedem Widerstands-Heizelement (41, 44) die Temperatur von einem jeweils zugehörigen Segment (15, 15.1, 15.2, 15.3, 15.4) der Trägerfläche (12) lokal einstellbar ist, und
- mit den Widerstands-Heizelementen (41, 44) eine Temperaturverteilung der Trägerfläche (12) einstellbar ist,
**gekennzeichnet durch**
- eine Vielzahl von Peltier-Kühlelementen (42, 45), die mit dem Substratkörper (11, 11.1) fest verbunden sind, wobei
- mit jedem Peltier-Kühlelement (42, 45) die Temperatur von einem jeweils zugehörigen Segment (15, 15.1, 15.2, 15.3, 15.4) der Trägerfläche (12) lokal einstellbar ist, und
- die Temperaturverteilung der Trägerfläche (12) mit den Peltier-Kühlelementen (42, 45) in Kombination mit den Widerstands-Heizelementen (41, 44) einstellbar ist.

2. Substrat gemäß Anspruch 1, mit mindestens einem der Merkmale:
- die Heizelemente (41, 44) und die Kühlelemente (42, 45) sind auf einer Rückseite des Substratkörpers (11, 11.1) entgegengesetzt zur Trägerfläche (12) angeordnet,
- die Heizelemente (41, 44) und die Kühlelemente (42, 45) sind in den Substratkörper (11, 11.1) eingebettet, und
- die Heizelemente (41, 44) und die Kühlelemente (42, 45) sind auf der Trägerfläche (12) und von dem thermoresponsiven Polymermaterial (13) bedeckt angeordnet.

3. Substrat gemäß einem der vorhergehenden Ansprüche, bei dem
- die Heizelemente (41, 44) und die Kühlelemente (42, 45) eine Matrixanordnung (43), eine Ringanordnung und/oder eine unregelmäßige Anordnung bilden.

4. Substrat gemäß einem der vorhergehenden Ansprüche, bei dem
- eine Elektrodeneinrichtung (50) vorgesehen ist, die mindestens ein Elektrodenelement (51) umfasst, mit dem oberhalb der Segmente (15, 15.1, 15.2, 15.3, 15.4) der Trägerfläche (12), deren Temperatur mit den Heizelementen (41, 44) und den Kühlelementen (42, 45) einstellbar ist, lokal wirkende, dielektrophoretische Kräfte generierbar sind.

5. Substrat gemäß einem der vorhergehenden Ansprüche, bei dem der Substratkörper (11, 11.1) mindestens eines der Merkmale umfasst:
- der Substratkörper (11, 11.1) hat eine Hohlform, auf deren Innenseite die Trägerfläche (12) gebildet ist,
- der Substratkörper (11, 11.1) ist mit einer gekrümmten Trägerfläche (12) gebildet,
- der Substratkörper (11, 11.1) umfasst eine Platte mit einer Vielzahl von Näpfen (14), deren innere Oberflächen die Trägerfläche (2.4) bilden, und
- der Substratkörper (11, 11.1) ist flüssigkeitsdurchlässig.

6. Kultivierungseinrichtung (30), die zur Kultivierung biologischer Zellen (20, 21, 22, 23, 24, 25) eingerichtet ist, die ein Substrat (10, 10.1) gemäß einem der vorhergehenden Ansprüche umfasst und zur Aufnahme eines die Trägerfläche (12) bedeckenden Kultivierungsmediums (34, 34.1, 34.2) eingerichtet ist.

7. Kultivierungseinrichtung gemäß Anspruch 6, die umfasst
- zwei Substrate (10, 10.1), deren Trägerflächen (12) einander gegenüberliegend mit einem Abstand angeordnet sind, so dass ein Kultivierungsraum zur Aufnahme des Kultivierungsmediums gebildet wird.

8. Kultivierungseinrichtung gemäß Anspruch 7, bei der
- das Substrat (10, 10.1) eine Hohlleitung umfasst, in der ein Kultivierungsraum zur Aufnahme des Kultivierungsmediums gebildet ist,
- ein fluidisches Mikrosystem (70), welches das Substrat (10, 10.1) und Fluidikelemente zur Leitung des Kultivierungsmediums umfasst, und/oder
- eine Vielzahl von Kultivierungsstationen (61, 62, 63, ...), zwischen denen das Substrat (10, 10.1, 10.2, 10.3) beweglich ist und die für die Einstellung verschiedener Kultivierungsbedingungen eingerichtet sind.

9. Kultivierungseinrichtung gemäß Anspruch 8, bei der
- an jeder der Kultivierungsstationen (61, 62, 63, ...) die Heizelemente und Kühlelemente (41, 42, 44, 45) angeordnet sind und das Substrat (10, 10.1, 10.2, 10.3) in thermischem Kontakt mit den Heizelementen und Kühlelementen (41, 42, 44, 45) steht, und/oder
- die Heizelemente und Kühlelemente (41, 42, 44, 45) mit dem Substrat (10, 10.1, 10.2, 10.3) beweglich und mit jeder der Kultivierungsstationen (61, 62, 63, ...) koppelbar sind.

10. Kultivierungseinrichtung gemäß Anspruch 8 oder 9, bei der
- das Substrat (10.2, 10.3) einen Substratstreifen umfasst, der für einen Durchlauf durch die Kultivierungsstationen (61, 62, 63, ...) eingerichtet ist.

11. Verfahren zur Anordnung biologischer Zellen (20, 21, 22, 23, 24, 25) auf einem Substrat (10, 10.1, 10.2, 10.3) gemäß einem der Ansprüche 1 bis 5 in einer Kultivierungseinrichtung gemäß einem der Ansprüche 6 bis 10, mit den Schritten:
- Einstellung der Temperatur der Segmente der Trägerfläche (12) unterhalb oder oberhalb einer Schalt-Temperatur des thermoresponsiven Polymermaterials (3), und
- Ablage der biologischen Zellen (20, 21, 22, 23, 24, 25) mit einem vorbestimmten Adhäsionsmuster auf den Segmenten, deren Temperatur oberhalb der Schalt-Temperatur des thermoresponsiven Polymermaterials (3) eingestellt ist.

12. Verfahren gemäß Anspruch 11, bei dem
- die Temperatur der Segmente der Trägerfläche (12) variiert wird, und bei der Ablage der biologischen Zellen (20, 21, 22, 23, 24, 25) das Adhäsionsmuster auf den Segmenten im Zeitverlauf verändert wird,
- die biologischen Zellen (20, 21, 22, 23, 24, 25) mindestens zwei verschiedene Zelltypen umfassen, die aufeinanderfolgend auf verschiedenen Segmenten abgelegt werden,
- eine Sammlung von suspendierten biologischen Zellen (21) im Kultivierungsmedium unter der Wirkung positiver Dielektrophorese an den Segmenten erfolgt, deren Temperatur oberhalb der Schalt-Temperatur des thermoresponsiven Polymermaterials (3) eingestellt ist, und/oder
- eine Ablösung und Abstoßung von biologischen Zellen (20, 21, 22) unter der Wirkung negativer Dielektrophorese von den Segmenten erfolgt, deren Temperatur unterhalb der Schalt-Temperatur des thermoresponsiven Polymermaterials (3) eingestellt ist.

13. Verfahren gemäß Anspruch 12, bei dem
- aufeinanderfolgend biologische Zellen (20, 21, 22, 23, 24, 25) mit verschiedenen Zelltypen in benachbarten Segmenten abgelegt und kultiviert werden.

14. Verfahren gemäß einem der Ansprüche 11 bis 13, bei dem
- zwei Substrate (10, 10.1) vorgesehen sind, deren Trägerflächen (12) einander gegenüberliegend mit einem Abstand angeordnet sind, so dass ein Kultivierungsraum zur Aufnahme des Kultivierungsmediums gebildet wird, mit den Schritten
- Einspülen des Kultivierungsmediums mit suspendierten biologischen Zellen in den Kultivierungsraum, und
- Einstellung der Temperatur der Segmente der Trägerflächen (12) derart, dass im Kultivierungsraum dreidimensionale Zellaggregate (25, 26) mit einer vorbestimmten Zusammensetzung gebildet werden.

15. Verfahren gemäß einem der Ansprüche 11 bis 14, mit dem Schritt
- Kultivierung der biologischen Zellen (20, 21, 22, 23, 24, 25) auf dem Substrat, insbesondere mit einer Nährstoffzufuhr durch den Substratkörper (11), und/oder
- Abkühlung von Segmenten unterhalb der Schalt-Temperatur des thermoresponsiven Polymermaterials (3) und Ablösung der biologischen Zellen von den abgekühlten Segmenten.

16. Verfahren gemäß einem der Ansprüche 11 bis 15, mit den Schritten
- Bewegung des mindestens einen Substrats (10, 10.1, 10.2, 10.3) zu einer Vielzahl von Kultivierungsstationen (61, 62, 63, ...), wobei
- an jeder der Kultivierungsstationen (61, 62, 63, ...) verschiedene Kultivierungsbedingungen vorgesehen ist.

## Claims

1. Substrate (10, 10.1, 10.2, 10.3) adapted for receiving biological cells (20, 21, 22, 23, 24, 25) in an adherent state, comprising:
- a substrate body (11, 11.1) with a carrier surface (12) on which a thermoresponsive polymer material (13) is arranged, and
- a plurality of resistance heating elements (41, 44) firmly connected to the substrate body (11, 11.1), wherein
- with each resistance heating element (41, 44), the temperature of an associated segment (15, 15.1, 15.2, 15.3, 15.4) of the carrier surface (12) is locally adjustable, and
- a temperature distribution over the carrier surface (12) is adjustable with the resistance heating elements (41, 44),
**characterized by**
- a plurality of Peltier cooling elements (42, 45) firmly connected to the substrate body (11, 11.1), wherein
- with each Peltier cooling element (42, 45), the temperature of an associated segment (15, 15.1, 15.2, 15.3, 15.4) of the carrier surface (12) is locally adjustable, and
- the temperature distribution over the carrier surface (12) is adjustable with the Peltier cooling elements (42, 45) in combination with the resistance heating elements (41, 44).

2. Substrate according to claim 1, having at least one of the following features:
- the heating elements (41, 44) and the cooling elements (42, 45) are arranged on a back of the substrate body (11, 11.1), opposite the carrier surface (12),
- the heating elements (41, 44) and the cooling elements (42, 45) are embedded in the substrate body (11, 11.1), and
- the heating elements (41, 44) and the cooling elements (42, 45) are arranged on the carrier surface (12) and are covered by the thermoresponsive polymer material (13).

3. Substrate according to one of the preceding claims, wherein
- the heating elements (41, 44) and the cooling elements (42, 45) form a matrix arrangement (43), an annular arrangement and/or an irregular arrangement.

4. Substrate according to one of the preceding claims, wherein
- an electrode device (50) is provided, comprising at least one electrode element (51) with which locally acting dielectrophoretic forces can be generated above the segments (15, 15.1, 15.2, 15.3, 15.4) of the carrier surface (12) whose temperature is adjustable with the heating elements (41, 44) and the cooling elements (42, 45).

5. Substrate according to one of the preceding claims, wherein the substrate body (11, 11.1) includes at least one of the following features:
- the substrate body (11, 11.1) has a hollow shape on the inside of which the carrier surface (12) is formed,
- the substrate body (11, 11.1) is formed with a curved carrier surface (12),
- the substrate body (11, 11.1) comprises a plate with a plurality of bowls (14) whose internal surfaces form the carrier surface (2.4), and
- the substrate body (11, 11.1) is permeable to liquids.

6. Cultivation device (30), provided for the culture of biological cells (20, 21, 22, 23, 24, 25), which comprises a substrate (10, 10.1) according to one of the preceding claims and is adapted for receiving a culture medium (34, 34.1, 34.2) that covers the carrier surface (12).

7. Cultivation device according to claim 6, which comprises
- two substrates (10, 10.1) whose carrier surfaces (12) are arranged opposite one another with a gap between them so as to form a cultivation volume for receiving the culture medium.

8. Cultivation device according to claim 7, wherein
- the substrate (10, 10.1) comprises a hollow duct forming a cultivation volume for receiving the culture medium,
- a fluidic microsystem (70) which comprises the substrate (10, 10.1) and fluidic elements for directing the culture medium, and/or
- a plurality of cultivation stations (61, 62, 63, ...) between which the substrate (10, 10.1, 10.2, 10.3) is moveable and which are provided for setting up different culture conditions.

9. Cultivation device according to claim 8, wherein
- the heating elements and cooling elements (41, 42, 44, 45) are arranged at each of the cultivation stations (61, 62, 63, ...) and the substrate (10, 10.1, 10.2, 10.3) is in thermal contact with the heating elements and cooling elements (41, 42, 44, 45), and/or
- the heating elements and cooling elements (41, 42, 44, 45), with the substrate (10, 10.1, 10.2, 10.3), are moveable and can be coupled with each of the cultivation stations (61, 62, 63, ...).

10. Cultivation device according to claim 8 or 9, wherein
- the substrate (10.2, 10.3) comprises a substrate strip provided for passage through the cultivation stations (61, 62, 63, ...).

11. Method of arranging biological cells (20, 21, 22, 23, 24, 25) on a substrate (10, 10.1, 10.2, 10.3) according to one of claims 1 to 5 in a cultivation device according to one of claims 6 to 10, comprising the following steps:
- setting the temperature of the segments of the carrier surface (12) below or above a switching temperature of the thermoresponsive polymer material (3), and
- depositing the biological cells (20, 21, 22, 23, 24, 25) in a predetermined adhesion pattern on the segments whose temperature is set above the switching temperature of the thermoresponsive polymer material (3).

12. Method according to claim 11, wherein
- the temperature of the segments of the carrier surface (12) is varied and, when the biological cells (20, 21, 22, 23, 24, 25) are deposited, the adhesion pattern on the segments is changed over time,
- the biological cells (20, 21, 22, 23, 24, 25) include at least two different cell types, which are successively deposited on different segments,
- suspended biological cells (21) are collected in the culture medium under the action of positive dielectrophoresis on the segments whose temperature is set above the switching temperature of the thermoresponsive polymer material (3), and/or
- biological cells (20, 21, 22) are detached and repelled under the action of negative dielectrophoresis from the segments whose temperature is set below the switching temperature of the thermoresponsive polymer material (3).

13. Method according to claim 12, wherein
- biological cells (20, 21, 22, 23, 24, 25) of different cell types are successively deposited in adjacent segments and cultured.

14. Method according to one of claims 11 to 13, wherein
- two substrates (10, 10.1) are provided whose carrier surfaces (12) are arranged opposite one another with a gap between them so as to form a cultivation volume for receiving the culture medium, comprising the following steps:
- flushing the culture medium with suspended biological cells into the cultivation volume, and
- setting the temperature of the segments of the carrier surfaces (12) in such a way as to form three-dimensional cell aggregates (25, 26) of predetermined composition in the cultivation volume.

15. Method according to one of claims 11 to 14, comprising the following step:
- culturing the biological cells (20, 21, 22, 23, 24, 25) on the substrate, especially with a supply of nutrient through the substrate body (11), and/or
- cooling segments below the switching temperature of the thermoresponsive polymer material (3) and detaching the biological cells from the cooled segments.

16. Method according to one of claims 11 to 15, comprising the following steps:
- moving at least one substrate (10, 10.1, 10.2, 10.3) to a plurality of cultivation stations (61, 62, 63, ...), wherein
- different culture conditions are provided at each of the cultivation stations (61, 62, 63, ...).

## Revendications

1. Substrat (10, 10.1, 10.2, 10.3), qui est mis au point pour recevoir des cellules biologiques (20, 21, 22, 23, 24, 25) dans l'état adhérent, comprenant :
- un corps de substrat (11, 11.1) pourvu d'une face porteuse (12), sur laquelle un matériau polymère (13) sensible à la température est disposé, et
- une pluralité d'éléments de chauffage par résistance (41, 44), qui sont reliés de manière solidaire au corps de substrat (11, 11.1), sachant que
- la température d'un segment (15, 15.1, 15.2, 15.3, 15.4) respectivement associé à la face porteuse (12) peut être réglée localement à l'aide de chaque élément de chauffage par résistance (41, 44), et
- une répartition de température de la face porteuse (12) peut être réglée à l'aide des éléments de chauffage par résistance (41, 44),
**caractérisé par**
- une pluralité d'éléments de refroidissement Peltier (42, 45), qui sont reliés de manière solidaire au corps de substrat (11, 11.1), sachant que
- la température d'un segment (15, 15.1, 15.2, 15.3, 15.4) respectivement associé à la face porteuse (12) peut être réglée localement à l'aide de chaque élément de refroidissement Peltier (42, 45), et
- la répartition de température de la face porteuse (12) peut être réglée à l'aide des éléments de refroidissement Peltier (42, 45) en combinaison avec les éléments de chauffage par résistance (41, 44).

2. Substrat selon la revendication 1, présentant au moins l'une des caractéristiques qui suit :
- les éléments de chauffage (41, 44) et les éléments de refroidissement (42, 45) sont disposés sur une face arrière du corps de substrat (11, 11.1) à l'opposé de la face porteuse (12),
- les éléments de chauffage (41, 44) et les éléments de refroidissement (42, 45) sont intégrés dans le corps de substrat (11, 11.1), et
- les éléments de chauffage (41, 44) et les éléments de refroidissement (42, 45) sont disposés sur la face porteuse (12) tout en étant couverts du matériau polymère (13) sensible à la température.

3. Substrat selon l'une quelconque des revendications précédentes, dans le cadre duquel
- les éléments de chauffage (41, 44) et les éléments de refroidissement (42, 45) forment un ensemble à matrices (43), un ensemble annulaire et/ou un ensemble irrégulier.

4. Substrat selon l'une quelconque des revendications précédentes, dans le cadre duquel
- un dispositif à électrodes (50) est prévu, lequel comprend au moins un élément formant électrode (51), lequel permet de générer des forces électrophorétiques agissant localement au-dessus des segments (15, 15.1, 15.2, 15.3, 15.4) de la face porteuse (12), dont la température peut être réglée à l'aide des éléments de chauffage (41, 44) et des éléments de refroidissement (42, 45).

5. Substrat selon l'une quelconque des revendications précédentes, dans le cadre duquel le corps de substrat (11, 11.1) présente au moins une des caractéristiques suivantes :
- le corps de substrat (11, 11.1) a une forme creuse, sur la face intérieure de laquelle est formée la face porteuse (12),
- le corps de substrat (11, 11.1) est formé avec une face porteuse (12) incurvée,
- le corps de substrat (11, 11.1) comprend une plaque pourvue d'une pluralité de coupelles (14), dont les surfaces intérieures forment la face porteuse (2.4), et
- le corps de substrat (11, 11.1) est perméable aux liquides.

6. Système de culture (30), qui est mis au point pour la culture de cellules biologiques (20, 21, 22, 23, 24, 25), lequel comprend un substrat (10, 10.1) selon l'une quelconque des revendications précédentes et qui est mis au point afin de recevoir un milieu de culture (34, 34.1, 34.2) couvrant la face porteuse (12).

7. Système de culture selon la revendication 6, qui comprend
- deux substrats (10, 10.1), dont les faces porteuses (12) sont disposées à une distance donnée de manière à se faire face les unes les autres de manière à former un espace de culture servant à recevoir le milieu de culture.

8. Système de culture selon la revendication 7, dans le cadre duquel
- le substrat (10, 10.1) comprend une conduite creuse, dans laquelle est formé un espace de culture servant à recevoir le milieu de culture,
- un microsystème (70) fluidique, qui comprend le substrat (10, 10.1) et les éléments fluidiques servant à acheminer le milieu de culture, et/ou
- une pluralité de postes de culture (61, 62, 63,...), entre lesquels le substrat (10, 10.1, 10.2, 10.3) est mobile et qui sont mis au point pour le réglage des différentes conditions de culture.

9. Système de culture selon la revendication 8, dans le cadre duquel
- les éléments de chauffage et les éléments de refroidissement (41, 42, 44, 45) sont disposés au niveau de chacun des postes de culture (61, 62, 63,...) et le substrat (10, 10.1, 10.2, 10.3) se trouve en contact thermique avec les éléments de chauffage et les éléments de refroidissement (41, 42, 44, 45), et/ou
- les éléments de chauffage et les éléments de refroidissement (41, 42, 44, 45) peuvent être couplés de manière mobile au substrat (10, 10.1, 10.2, 10.3) et à chacun des postes de culture (61, 62, 63,...).

10. Système de culture selon la revendication 8 ou 9, dans le cadre duquel
- le substrat (10.2, 10.3) comprend une bande de substrat, qui est mise au point en vue d'un passage à travers les postes de culture (61, 62, 63, ...) .

11. Procédé servant à disposer des cellules biologiques (20, 21, 22, 23, 24, 25) sur un substrat (10, 10.1, 10.2, 10.3) selon l'une quelconque des revendications 1 à 5 dans un système de culture selon l'une quelconque des revendications 6 à 10, comprenant les étapes suivantes consistant à :
- régler la température des segments de la face porteuse (12) à une température inférieure ou supérieure à une température d'activation du matériau polymère (3) sensible à la température, et
- déposer les cellules biologiques (20, 21, 22, 23, 24, 25) à l'aide d'un modèle d'adhésion prédéfini, sur les segments, dont la température est réglée à une température supérieure à la température d'activation du matériau polymère (3) sensible à la température.

12. Procédé selon la revendication 11, dans le cadre duquel
- la température des segments de la face porteuse (12) varie, et le modèle d'adhésion sur les segments est modifié au fil du temps lors du dépôt des cellules biologiques (20, 21, 22, 23, 24, 25),
- les cellules biologiques (20, 21, 22, 23, 24, 25) comprennent au moins deux types de cellules différents, qui sont déposés les uns sur les autres sur différents segments,
- une accumulation de cellules biologiques (21) en suspension dans le milieu de culture sous l'action d'une diélectrophorèse positive sur les segments est effectuée, dont la température est réglée à une température supérieure à la température d'activation du matériau polymère (3) sensible à la température, et/ou
- un détachement et un rejet des cellules biologiques (20, 21, 22) sous l'action d'une diélectrophorèse négative des segments sont effectués, dont la température est réglée à une température inférieure à la température d'activation du matériau polymère (3) sensible à la température.

13. Procédé selon la revendication 12, dans le cadre duquel,
- des cellules biologiques (20, 21, 22, 23, 24, 25) présentant différents types de cellule sont déposées les unes sur les autres et cultivées dans des segments adjacents.

14. Procédé selon l'une quelconque des revendications 11 à 13, dans le cadre duquel
- deux substrats (10, 10.1) sont prévus, dont les faces porteuses (12) sont disposées à une distance donnée de manière à se faire face les unes les autres, de manière à former un espace de culture servant à recevoir le milieu de culture, comprenant les étapes suivantes consistant à :
- injecter le milieu de culture comprenant des cellules biologiques en suspension dans l'espace de culture, et
- régler la température des segments des faces porteuses (12) de manière à former, dans l'espace de culture, des agrégats de cellules (25, 26) tridimensionnels présentant une composition prédéfinie.

15. Procédé selon l'une quelconque des revendications 11 à 14, comprenant l'étape consistant à
- cultiver les cellules biologiques (20, 21, 22, 23, 24, 25) sur le substrat, en particulier à l'aide d'un apport en substances nutritives par le corps de substrat (11), et/ou
- refroidir des segments à une température inférieure à la température d'activation du matériau polymère (3) sensible à la température et détacher les cellules biologiques des segments refroidis.

16. Procédé selon l'une quelconque des revendications 11 à 15, comprenant les étapes suivantes consistant à :
- déplacer le substrat (10, 10.1, 10.2, 10.3) au moins au nombre de un pour obtenir une pluralité de postes de culture (61, 62, 63,...), sachant que
- différentes conditions de culture sont prévues sur chacun des postes de culture (61, 62, 63, ...).
